# EUROPEAN PATENT APPLICATION

(11) **EP 4 273 237 A1**
(43) Date of publication of application: **08.11.2023**
(21) Application number: 21924110.6
(22) Date of filing: 06.07.2021
(51) Int. Cl.: C12N 9/10, C12N 15/54, C12P 13/00, C12P 17/12, C12P 17/14

(54) **TRANSAMINASE MUTANT AND APPLICATION THEREOF**

(30) Priority: 04.02.2021 CN 202110150760; 26.04.2021 CN 202110451381
(71) Applicant: Asymchem Laboratories (Tianjin) Co., Ltd., Tianjin 300457 (CN)
(72) Inventor: HONG, Hao, Morrisville, North Carolina 27560 (US); JAMES, Gage, Morrisville, North Carolina 27560 (US); XIAO, Yi, Tianjin 300457 (CN); ZHANG, Na, Tianjin 300457 (CN); JIAO, Xuecheng, Tianjin 300457 (CN); MA, Yulei, Tianjin 300457 (CN); MOU, Huiyan, Tianjin 300457 (CN); WANG, Zujian, Tianjin 300457 (CN); SUN, Kaihua, Tianjin 300457 (CN); LI, Xiang, Tianjin 300457 (CN); ZHAO, Tong, Tianjin 300457 (CN); CAO, Shan, Tianjin 300457 (CN)
(74) Representative: Studio Torta S.p.A.
(86) International application number: PCT/CN2021/104841
(87) International publication number: WO 2022/166103

(57) **Abstract**

Provided are transaminase mutants and uses thereof. The transaminase mutant is obtained by one or more amino acid mutations occurring in SEQ ID NO: 2 or is a mutant with a conserved amino acid mutation obtained by taking the sequence SEQ ID NO: 1 of a wild-type CvTA transaminase as a reference. Compared with wild-type transaminases, the catalytic activity of the mutant is improved to different degrees, so that the production efficiency of chiral amine compound synthesis may be improved.

## Description

### Technical Field

The present disclosure relates to the field of biological enzyme applications, in particular to transaminase mutants and uses thereof.

### Background

Large sterically hindered chiral amine compounds (in the present disclosure, it refers to a class of compounds with a group next to a latent chiral carbonyl that is greater than a methyl) are a class of optically active substances and functional molecules, and are important chiral intermediates widely used in drug and ligand synthesis. However, there are currently few reports on synthesis of such chiral compounds, and satisfactory results may not be achieved. For example, the selectivity of phenyl tert-butyl amino ester with a large steric hindrance on one side of the carbonyl is 76% (Angelw. Chem. Int. Ed. 2007, 46, 4367). At present, some metal catalysts, such as ruthenium, rhodium, and palladium, all have the better stereoselectivity reported for an asymmetric hydrogenation reaction with a small steric hindrance (in the present disclosure, it refers to a class of compounds with a group next to the latent chiral carbonyl that is H or methyl). However, as the approached steric hindrance of the carbonyl becomes larger, the catalytic effect becomes worse.

The asymmetric hydrogenation is a type of technologies for production of fine chemicals and drug precursors. However, this technology has some drawbacks, such as the need to use a high-pressure hydrogen gas, which poses a great challenge to production safety and requires expensive transition metal catalysts. These toxic catalysts also need to be carefully treated and removed in the post-treatment process. In addition, this process requires a lot of human and material resources to screen corresponding ligands. In many cases, there is still a problem of low stereoselectivity in this process (Science, 2010, 329, 305).

The asymmetric transamination catalyzed by transaminases provides an economical and green way for the synthesis of the chiral amine compounds. However, the transaminase naturally has two pockets, namely, one large and one small, in structure, so the substrate that it may convert has greater limitations. At present, the transaminase with catalytic activity for the chiral amine compounds with the large steric hindrance is not found yet. In general, if the group next to the latent chiral carbonyl is larger than the methyl, the small pocket of the transaminase is unacceptable. Therefore, the synthesis of the chiral amine compounds with the large steric hindrance catalyzed by the transaminase is a long-standing challenge.

### Summary

A main purpose of the present disclosure is to provide transaminase mutants and uses thereof, as to solve a problem in prior art that a transaminase is difficult to catalyze synthesis of a large sterically hindered chiral amine compound.

In order to achieve the above purpose, according to one aspect of the present disclosure, a transaminase mutant is provided, and the transaminase mutant is selected from: (1) a mutant obtained from a mutation of amino acids of SEQ ID NO: 2, wherein the mutation of amino acids is any one of the group consisting of: F22A, L59A, W60A, C61A, F88A, Y89A, K90A, T107A, N151A, Y153A, F166A, E168A, E171A, K193A, V234A, I262A, I297A, K304A, F320A, T321A, E368A, L379A, L380A, F397A, R405A, F409A, D416A, S417A, C418A, and S424A; or (2) a mutant obtained from a mutation of amino acids of SEQ ID NO: 2, wherein the mutation of amino acids is F88A and any one or more of mutations in the following positions: L59, P83, F84, Y85, N86, T87, Y89, K90, T91, F409, and A417; or (3) a mutant obtained from a mutation of conserved amino acids of a wild-type transaminase, wherein the conserved amino acids are located at positions 54 to 63 and positions 84 to 96 of the SEQ ID NO: 1 with the sequence SEQ ID NO: 1 of the wild-type CvTA transaminase as a reference, and the conserved amino acid undergoes at least any one of the following mutations: L59A, W60A, C61A, F84S/C/G/UR/V/P/A/M/E/K/W/Q/T/H/D/Y/N/I, Y85M, N86H/Q/P/D/K/Y/S/UM/I/T/W/F/V/R/S/K/G, T87K/C/E/N/Q/H/F/R/D/I/M/W/A/P/V/S/UG, F88A, Y89D/A/, K90A/F/G/D/C/S/A/E/UV/I/R/T/Y/M/H/N/P/Q/W, and T91 M/W/V/G/C/R/A/F/I/M/E/L/S/K/H/Q/D/N/P/Y, wherein "/" represents "or".

Further, in (3), the wild-type transaminase is a transaminase having 69% or more, preferably 75% or more, more preferably 80% or more, and further preferably 85% or more identity with the SEQ ID NO:1, and having the conserved amino acids; preferably, the wild-type transaminase is selected from any one of the sources of enzyme numbers 1 to 54 shown in Table 9 and Table 11.

Further, in (3), the conserved amino acid undergoes at least any one of the following combined mutations: (a) L59A+F88A; (b) L59A+F88A+F89D; (c) L59A+F88A+F89D+N86H; and (d) L59A+F88A+F89D+N86H+Y85M.

Further, in (1) or (2), the amino acid mutation is any one of the following:

| | | |
|---|---|---|
| F22A | F88A+L59H | F88A+L59A+Y89C |
| L59A | F88A+L59R | F88A+L59A+Y89T |
| F88A | F88A+L59F | F88A+L59A+Y89K |
| Y89A | F88A+L59Y | F88A+L59A+Y89D+T87G |
| K90A | F88A+L59S | F88A+L59A+Y89D+T87L |
| T107A | F88A+L59C | F88A+L59A+Y89D+T87S |
| N151A | F88A+L59Q | F88A+L59A+Y89D+T87V |
| Y153A | F88A+L59M | F88A+L59A+Y89D+T87P |
| F166A | F88A+L59L | F88A+L59A+Y89D+T87A |
| E171A | F88A+L59V | F88A+L59A+Y89D+T87W |
| K193A | F88A+L59G | F88A+L59A+Y89D+T87M |
| V234A | F88A+L59A | F88 A+L59A+Y89D+T87I |
| I262A | F88A+L59D | F88A+L59A+Y89D+T87D |
| I297A | F88A+L59N | F88A+L59A+Y89D+T87R |
| K304A | F88A+L59R | F88A+L59A+Y89D+T87F |
| F320A | F88A+L59W | F88A+L59A+Y89D+T87H |
| T321A | F88A+L59P | F88A+L59A+Y89D+T87Q |
| E368A | F88A+L59A+Y89G | F88A+L59A+Y89D+T87N |
| E368A | F88A+L59A+Y89L | F88A+L59A+Y89D+T87E |
| L380A | F88A+L59A+Y89S | F88A+L59A+Y89D+T87C |
| F397A | F88A+L59A+Y89V | F88A+L59A+Y89D+T87K |
| R405A | F88A+L59A+Y89P | F88A+L59A+Y89D+N86G |
| F409A | F88A+L59A+Y89A | F88A+L59A+Y89D+N86C |
| D416A | F88A+L59A+Y89W | F88A+L59A+Y89D+N86S |
| S417A | F88A+L59A+Y89M | F88A+L59A+Y89D+N86R |
| C418A | F88A+L59A+Y89I | F88A+L59A+Y89D+N86V |
| S424A | F88A+L59A+Y89D | F88A+L59A+Y89D+N86F |
| / | F88A+L59A+Y89R | F88A+L59A+Y89D+N86W |
| / | F88A+L59A+Y89F | F88A+L59A+Y89D+N86T |
| / | F88A+L59A+Y89H | F88A+L59A+Y89D+N86I |
| / | F88A+L59A+Y89Q | F88A+L59A+Y89D+N86M |
| / | F88A+L59A+Y89N | F88A+L59A+Y89D+N86L |
| / | F88A+L59A+Y89E | F88A+L59A+Y89D+N86A |
| F88A+L59A+Y89D+N86E | F88A+L59A+Y89D+N86Q | F88A+L59A+Y89D+N86Y |
| | F88A+L59A+Y89D+N86P | F88A+L59A+Y89D+N86K |
| | F88A+L59A+Y89D+N86H | F88A+L59A+Y89D+N86D |

| | |
|---|---|
| F88A+L59A+Y89D+N86H+F84S | F88A+L59A+Y89D+N86H+Y85M+K90I |
| F88A+L59A+Y89D+N86H+F84C | F88A+L59A+Y89D+N86H+Y85M+K90R |
| F88A+L59A+Y89D+N86H+F84G | F88A+L59A+Y89D+N86H+Y85M+K90T |
| F88A+L59A+Y89D+N86H+F84L | F88A+L59A+Y89D+N86H+Y85M+K90Y |
| F88A+L59A+Y89D+N86H+F84R | F88A+L59A+Y89D+N86H+Y85M+K90M |
| F88A+L59A+Y89D+N86H+F84V | F88A+L59A+Y89D+N86H+Y85M+K90H |
| F88A+L59A+Y89D+N86H+F84P | F88A+L59A+Y89D+N86H+Y85M+K90N |
| F88A+L59A+Y89D+N86H+F84A | F88A+L59A+Y89D+N86H+Y85M+K90P |
| F88A+L59A+Y89D+N86H+F84M | F88A+L59A+Y89D+N86H+Y85M+K90Q |
| F88A+L59A+Y89D+N86H+F84E | F88A+L59A+Y89D+N86H+Y85M+K90W |
| F88A+L59A+Y89D+N86H+F84K | F88A+L59A+Y89D+N86H+Y85M+T91W |
| F88A+L59A+Y89D+N86H+F84W | F88A+L59A+Y89D+N86H+Y85M+T91V |
| F88A+L59A+Y89D+N86H+F84Q | F88A+L59A+Y89D+N86H+Y85M+T91G |
| F88A+L59A+Y89D+N86H+F84T | F88A+L59A+Y89D+N86H+Y85M+T91C |
| F88A+L59A+Y89D+N86H+F84H | F88A+L59A+Y89D+N86H+Y85M+T91R |
| F88A+L59A+Y89D+N86H+F84D | F88A+L59A+Y89D+N86H+Y85M+T91A |
| F88A+L59A+Y89D+N86H+F84Y | F88A+L59A+Y89D+N86H+Y85M+T91F |
| F88A+L59A+Y89D+N86H+F84N | F88A+L59A+Y89D+N86H+Y85M+T91I |
| F88A+L59A+Y89D+N86H+F84I | F88A+L59A+Y89D+N86H+Y85M+T91M |
| F88A+L59A+Y89D+N86H+Y85M | F88A+L59A+Y89D+N86H+Y85M+T91E |
| F88A+L59A+Y89D+N86H+Y85M+K90F | F88A+L59A+Y89D+N86H+Y85M+T91L |
| F88A+L59A+Y89D+N86H+Y85M+K90G | F88A+L59A+Y89D+N86H+Y85M+T91S |
| F88A+L59A+Y89D+N86H+Y85M+K90D | F88A+L59A+Y89D+N86H+Y85M+T91K |
| F88A+L59A+Y89D+N86H+Y85M+K90C | F88A+L59A+Y89D+N86H+Y85M+T91H |
| F88A+L59A+Y89D+N86H+Y85M+K90S | F88A+L59A+Y89D+N86H+Y85M+T91Q |
| F88A+L59A+Y89D+N86H+Y85M+K90A | F88A+L59A+Y89D+N86H+Y85M+T91D |
| F88A+L59A+Y89D+N86H+Y85M+K90E | F88A+L59A+Y89D+N86H+Y85M+T91N |
| F88A+L59A+Y89D+N86H+Y85M+K90L | F88A+L59A+Y89D+N86H+Y85M+T91P |
| F88A+L59A+Y89D+N86H+Y85M+K90V | F88A+L59A+Y89D+N86H+Y85M+T91Y |

| | |
|---|---|
| F88A+L59A+Y89D+N86H+Y85M+K90G+T91M | F88A+L59A+Y89D+N86H+Y85M+K90G+T91 M+F409W |
| F88A+L59A+Y89D+N86H+Y85M+K90G+T91M+P83F | F88A+L59A+Y89D+N86H+Y85M+K90G+T91M+F409N |
| F88A+L59A+Y89D+N86H+Y85M+K90G+T91M+P83G | F88A+L59A+Y89D+N86H+Y85M+K90G+T91M+S417F |
| F88A+L59A+Y89D+N86H+Y85M+K90G+T91M+P83D | F88A+L59A+Y89D+N86H+Y85M+K90G+T91M+S417E |
| F88A+L59A+Y89D+N86H+Y85M+K90G+T91M+P83C | F88A+L59A+Y89D+N86H+Y85M+K90G+T91M+S417Y |
| F88A+L59A+Y89D+N86H+Y85M+K90G+T91M+P83S | F88A+L59A+Y89D+N86H+Y85M+K90G+T91M+S417M |
| F88A+L59A+Y89D+N86H+Y85M+K90G+T91M+P83A | F88A+L59A+Y89D+N86H+Y85M+K90G+T91M+S417A |
| F88A+L59A+Y89D+N86H+Y85M+K90G+T91M+P83E | F88A+L59A+Y89D+N86H+Y85M+K90G+T91M+S417Q |
| F88A+L59A+Y89D+N86H+Y85M+K90G+T91M+P83L | F88A+L59A+Y89D+N86H+Y85M+K90G+T91M+S417C |
| F88A+L59A+Y89D+N86H+Y85M+K90G+T91M+P83V | F88A+L59A+Y89D+N86H+Y85M+K90G+T91M+S417L |
| F88A+L59A+Y89D+N86H+Y85M+K90G+T91 M+P83I | F88A+L59A+Y89D+N86H+Y85M+K90G+T91M+S417I |
| F88A+L59A+Y89D+N86H+Y85M+K90G+T91M+P83R | F88A+L59A+Y89D+N86H+Y85M+K90G+T91M+S417T |
| F88A+L59A+Y89D+N86H+Y85M+K90G+T91M+P83T | F88A+L59A+Y89D+N86H+Y85M+K90G+T91M+S417V |
| F88A+L59A+Y89D+N86H+Y85M+K90G+T91M+P83Y | F88A+L59A+Y89D+N86H+Y85M+K90G+T91 M+S417W |
| F88A+L59A+Y89D+N86H+Y85M+K90G+T91 M+P83M | F88A+L59A+Y89D+N86H+Y85M+K90G+T91M+S424N |
| F88A+L59A+Y89D+N86H+Y85M+K90G+T91M+P83H | F88A+L59A+Y89D+N86H+Y85M+K90G+T91M+S424F |
| F88A+L59A+Y89D+N86H+Y85M+K90G+T91M+P83N | F88A+L59A+Y89D+N86H+Y85M+K90G+T91M+S424T |
| F88A+L59A+Y89D+N86H+Y85M+K90G+T91M+P83K | F88A+L59A+Y89D+N86H+Y85M+K90G+T91M+S424G |
| F88A+L59A+Y89D+N86H+Y85M+K90G+T91M+P83Q | F88A+L59A+Y89D+N86H+Y85M+K90G+T91 M+S424I |
| F88A+L59A+Y89D+N86H+Y85M+K90G+T91 M+P83W | F88A+L59A+Y89D+N86H+Y85M+K90G+T91M+S424V |
| F88A+L59A+Y89D+N86H+Y85M+K90G+T91M+F409Y | F88A+L59A+Y89D+N86H+Y85M+K90G+T91M+S424A |
| F88A+L59A+Y89D+N86H+Y85M+K90G+T91 M+F409I | F88A+L59A+Y89D+N86H+Y85M+K90G+T91M+S424L |
| F88A+L59A+Y89D+N86H+Y85M+K90G+T91M+F409G | F88A+L59A+Y89D+N86H+Y85M+K90G+T91M+V234A |
| F88A+L59A+Y89D+N86H+Y85M+K90G+T91M+F409F | F88A+L59A+Y89D+N86H+Y85M+K90G+T91M+L380A |
| F88A+L59A+Y89D+N86H+Y85M+K90G+T91M+F409A | F88A+L59A+Y89D+N86H+Y85M+K90G+T91M+C61A |
| F88A+L59A+Y89D+N86H+Y85M+K90G+T91M+F409L | F88A+L59A+Y89D+N86H+Y85M+K90G+T91M+P83S |

| |
|---|
| F88A+L59A+Y89D+N86H+Y85M+K90G+T91M+P83S+V234A |
| F88A+L59A+Y89D+N86H+Y85M+K90G+T91M+P83S+V234A+L380A |
| F88A+L59A+Y89D+N86H+Y85M+K90G+T91M+P83S+V234A+L380A+S417A |
| F88A+L59A+Y89D+N86H+Y85M+K90G+T91M+P83S+V234A+L380A+S4170 |
| F88A+L59A+Y89D+N86H+Y85M+K90G+T91M+P83S+V234A+L380A+S417D |
| F88A+L59A+Y89D+N86H+Y85M+K90G+T91M+P83S+V234A+L380A+S417E |
| F88A+L59A+Y89D+N86H+Y85M+K90G+T91M+P83S+V234A+L380A+S417F |
| F88A+L59A+Y89D+N86H+Y85M+K90G+T91M+P83S+V234A+L380A+S417G |
| F88A+L59A+Y89D+N86H+Y85M+K90G+T91M+P83S+V234A+L380A+S417H |
| F88A+L59A+Y89D+N86H+Y85M+K90G+T91M+P83S+V234A+L380A+S417I |
| F88A+L59A+Y89D+N86H+Y85M+K90G+T91M+P83S+V234A+L380A+S417K |
| F88A+L59A+Y89D+N86H+Y85M+K90G+T91M+P83S+V234A+L380A+S417L |
| F88A+L59A+Y89D+N86H+Y85M+K90G+T91M+P83S+V234A+L380A+S417M |
| F88A+L59A+Y89D+N86H+Y85M+K90G+T91M+P83S+V234A+L380A+S417N |
| F88A+L59A+Y89D+N86H+Y85M+K90G+T91M+P83S+V234A+L380A+S417P |
| F88A+L59A+Y89D+N86H+Y85M+K90G+T91M+P83S+V234A+L380A+S417Q |
| F88A+L59A+Y89D+N86H+Y85M+K90G+T91M+P83S+V234A+L380A+S417R |
| F88A+L59A+Y89D+N86H+Y85M+K90G+T91M+P83S+V234A+L380A+S417T |
| F88A+L59A+Y89D+N86H+Y85M+K90G+T91M+P83S+V234A+L380A+S417V |
| F88A+L59A+Y89D+N86H+Y85M+K90G+T91M+P83S+V234A+L380A+S417W |
| F88A+L59A+Y89D+N86H+Y85M+K90G+T91M+P83S+V234A+L380A+S417Y |
| F88A+L59A+Y89D+N86H+Y85M+K90G+T91M+P83S+V234A+L380A+S417A+F409L |
| F88A+L59A+Y89D+N86H+Y85M+K90G+T91M+P83S+V234A+L380A+S417A+S424A |
| F88A+L59A+Y89D+N86H+Y85M+K90G+T91M+P83S+V234A+L380A+S417A+S424G |
| F88A+L59A+Y89D+N86H+Y85M+K90G+T91M+P83S+V234A+L380A+S417A+K167I |
| F88A+L59A+Y89D+N86H+Y85M+K90G+T91M+P83S+V234C+L380A+S417A |
| F88A+L59A+Y89D+N86H+Y8SM+K90G+T91 M+P83S+V234A+L380A+S417I+A168E |
| F88A+L59A+Y89D+N86H+Y85M+K90G+T91M+P3S+V234A+L380A+S417I+A168E+A379L+S424A |
| F88A+L59A+Y89D+N86H+Y85M+K90G+T91M+P3S+V234A+L380A+S417I+A168E+A379L+S424G |
| F88A+L59A+Y89D+N86H+Y85M+K90G+T91M+P3S+V234C+L380A+S417I+A168E+A379L+S424A |
| F88A+L59A+Y89D+N86H+Y85M+K90G+T91M+P3S+V234A+L380A+S417I+A168E+A379L+S424A+F 301S |
| F88A+L59A+Y89D+N86H+Y85M+K90G+T91M+P3S+V234A+L380A+S417I+A168E+A379L+S424A+F 364C |
| F88A+L59A+Y89D+N86H+Y85M+K90G+T91M+P3S+V234A+L380A+S417I+A168E+A379L+S424A+T 452A |
| F88A+L59A+Y89D+N86H+Y85M+K90G+T91M+P3S+V234A+L380A+S417I+A168E+A379L+S424A+K 358R+F449L |
| F88A+L59A+Y89D+N86H+Y85M+K90G+T91M+P3S+V234A+L380A+S417I+A168E+A379L+S424A+M 180V |
| F88A+L59A+Y89D+N86H+Y85M+K90G+T91M+P3S+V234A+L380A+S417I+A168E+A379L+S424A+G 164S |
| F88A+L59A+Y89D+N86H+Y85M+K90G+T91M+P3S+V234A+L380A+S417I+A168E+A379L+S424A+F 301S+G164S |
| F88A+L59A+Y89D+N86H+Y85M+K90G+T91M+P3S+V234A+L380A+S417I+A168E+A379L+S424A+F 301S+M180V |
| F88A+L59A+Y89D+N86H+Y85M+K90G+T91M+P3S+V234A+L380A+S417I+A168E+A379L+S424A+F 301S+F449L |
| F88A+L59A+Y89D+N86H+Y85M+K90G+T91M+P3S+V234A+L380A+S417I+A168E+A379L+S424A+F 301S+F409L |
| F88A+L59A+Y89D+N86H+Y85M+K90G+T91M+P3S+V234A+L380A+S417I+A168E+A379L+S424A+F 301S+G164S+T452S |
| F88A+L59A+Y89D+N86H+Y85M+K90G+T91M+P3S+V234A+L380A+S417I+A168E+A379L+S424A+F 301S+G164S+F449L |
| F88A+L59A+Y89D+N86H+Y85M+K90G+T91M+P3S+V234A+L380A+S417I+A168E+A379L+S424A+F 301S+G164S+M180V |
| F88A+L59A+Y89D+N86H+Y85M+K90G+T91M+P3S+V234A+L380A+S417I+A168E+A379L+S424A+F 301S+G164S+M180V+E171V |
| F88A+L59A+Y89D+N86H+Y85M+K90G+T91M+P3S+V234A+L380A+S417I+A168E+A379L+S424A+F 301S+G164S+F449L+L453V |
| F88A+L59A+Y89D+N86H+Y85M+K90G+T91M+P3S+V234A+L380A+S417I+A168E+A379L+S424A+F 301S+G164S+K167I |
| F88A+L59A+Y89D+N86H+Y85M+K90G+T91M+P3S+V234A+L380A+S417I+A168E+A379L+S424A+F 301S+G164S+T452S+M180V |
| F88A+L59A+Y89D+N86H+Y85M+K90G+T91M+P3S+V234A+L380A+S417I+A168E+A379L+S424A+F 301S+G164S+T452S+F449L |
| F88A+L59A+Y89D+N86H+Y85M+K90G+T91M+P3S+V234A+L380A+S417I+A168E+A379L+S424A+F 301S+G164S+T452S+M180V+E171V |
| F88A+L59A+Y89D+N86H+Y85M+K90G+T91M+P3S+V234A+L380A+S417I+A168E+A379L+S424A+F 301S+G164S+T452S+M180V+F449L |
| F88A+L59A+Y89D+N86H+Y85M+K90G+T91M+P3S+V234A+L380A+S417I+A168E+A379L+S424A+F 301S+G164S+T452S+M180V+F320M |
| F88A+L59A+Y89D+N86H+Y85M+K90G+T91M+P3S+V234A+L380A+S417I+A168E+A379L+S424A+F 301S+G164S+T452S+M180V+F320H |
| F88A+L59A+Y89D+N86H+Y85M+K90G+T91M+P3S+V234A+L380A+S417I+A168E+A379L+S424A+F 301S+G164S+T452S+M180V+Y322N |
| F88A+L59A+Y89D+N86H+Y85M+K90G+T91M+P3S+V234A+L380A+S417I+A168E+A379L+S424A+F 301S+G164S+T452S+M180V+Y322T |
| F88A+L59A+Y89D+N86H+Y85M+K90G+T91M+P3S+V234A+L380A+S417I+A168E+A379L+S424A+F 301S+G164S+T452S+M180V+F449L+F320H+Y322C |
| F88A+L59A+Y89D+N86H+Y85M+K90G+T91M+P3S+V234A+L380A+S417I+A168E+A379L+S424A+F 301S+G164S+T452S+M180V+F449L+F320H+Y322T |
| F88A+L59A+Y89D+N86H+Y85M+K90G+T91M+P3S+V234A+L380A+S417I+A168E+A379L+S424A+F |
| 301S+G164S+T452S+M180V+F449L+F320H+Y322S |
| F88A+L59A+Y89D+N86H+Y85M+K90G+T91M+P3S+V234A+L380A+S417I+A168E+A379L+S424A+F 301S+G164S+T452S+M180V+F449L+Y322S |
| F88A+L59A+Y89D+N86H+Y85M+K90G+T91M+P3S+V234A+L380A+S417I+A168E+A379L+S424A+F 301S+G164S+T452S+M180V+F449L+Y322T |
| F88A+L59A+Y89D+N86H+Y85M+K90G+T91M+P3S+V234A+L380A+S417I+A168E+A379L+S424A+F 301S+G164S+T452S+M180V+F449L+F320H+Y322T+M911 |
| F88A+L59A+Y89D+N86H+Y85M+K90G+T91M+P3S+V234A+L380A+S417I+A168E+A379L+S424A+F 301S+G164S+T452S+M180V+F449L+F320H+Y322T+D315V |

In order to achieve the above purpose, according to a second aspect of the present disclosure, a DNA molecule is provided, and the DNA molecule encodes any one of the above transaminase mutants.

In order to achieve the above purpose, according to a third aspect of the present disclosure, a recombinant plasmid is provided, and the recombinant plasmid is ligated with the above DNA molecule.

In order to achieve the above purpose, according to a fourth aspect of the present disclosure, a synthesis method for a chiral amine compound is provided, and any one of the above transaminase mutants is used to transaminate a ketone substrate shown in Formula I under the action of an amino donor, to obtain the chiral amine compound;

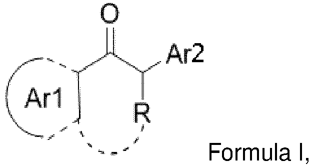

Ar1 represents a substituted or unsubstituted aryl, or a substituted or unsubstituted arylene, or a substituted or unsubstituted heteroarylene; Ar2 represents a substituted or unsubstituted aryl or a substituted or unsubstituted cycloalkyl; and R is optional, herein R represents a substituted or unsubstituted C1~C5 alkylene, and R is connected to Ar1 or Ar2 to form a ring; herein, a substituent in the substituted aryl, the substituted aryl arylene, the substituted heteroarylene or the substituted alkylene is independently selected from halogen, hydroxy, or amino, and a heteroatom in the substituted heteroarylene is selected from N, O or S.

Further, the substituent in the substituted aryl or arylene is halogen or -S-CH3, the halogen or -S-CH₃ is located at any one or more positions in an ortho-position, a meta-position, or a para-position of the aryl or arylene, and preferably, the halogen is F, Cl, or Br.

Further, Ar1 represents a substituted or unsubstituted aryl or arylene, Ar2 represents an unsubstituted aryl, R represents an unsubstituted C1-C5 alkylene, and R is connected to Ar1 to form a ring.

Further, Ar1 represents an unsubstituted heteroarylene, Ar2 represents a halogen-substituted aryl, R represents a hydroxyl-substituted C1-C5 alkylene, and R is connected to Ar1 to form a ring.

Further, Ar1 represents an arylene substituted by halogen and amino group, and Ar2 represents a C3-C8 cycloalkyl.

Further, Ar1 represents an unsubstituted cycloalkyl or aryl, and Ar2 represents a substituted or unsubstituted cycloalkyl or aryl.

Further, Ar1 represents a hydroxyl, a methyl, an ethyl, or a -S-CH₃-substituted cycloalkyl or aryl, and Ar2 represents an unsubstituted aryl or alkyl.

Further, the ketone substrate is:

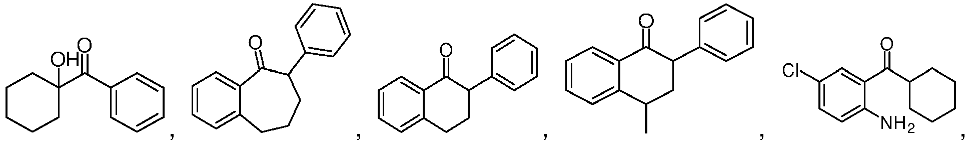

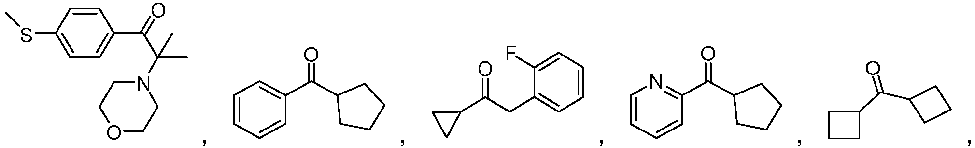

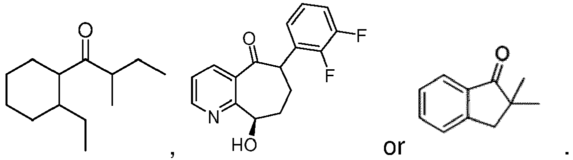

Further, the amino donor is isopropylamine, isopropylamine hydrochloride, alanine, n-butylamine, or aniline.

By applying technical schemes of the present disclosure, a class of transaminases with certain catalytic activity for the specific substrate is screened, and it is analyzed that such transaminases have conserved amino acid sequence regions. By site-directed mutation and directional screening of the amino acids in the conserved regions, a series of transaminase mutants with improved catalytic activity than the wild type is obtained. The use of these mutants for the synthesis of the chiral amine compounds, especially for the synthesis of large sterically hindered chiral amines, is beneficial for improving the production efficiency and reducing the industrial production cost. The use of biological enzymes for green chemical synthesis is beneficial for reducing industrial three wastes.

### Brief Description of the Drawings

Drawings of the description for constituting a part of the present disclosure are used to provide further understanding of the present disclosure. Schematic embodiments of the present disclosure and descriptions thereof are used to explain the present disclosure, and do not constitute improper limitation to the present disclosure. In the drawings:
Fig. 1 shows a schematic diagram of partial results of multi-sequence (SEQ ID NO: 1 and SEQ ID NO: 3 to SEQ ID NO: 20) alignment according to an embodiment of the present disclosure;
Fig. 2 shows a schematic diagram of partial results of multi-sequence (SEQ ID NO: 20 to SEQ ID NO: 38) alignment according to an embodiment of the present disclosure; and
Fig. 3 shows a schematic diagram of partial results of multi-sequence (SEQ ID NO: 39 to SEQ ID NO: 56) alignment according to an embodiment of the present disclosure.

### Detailed Description of the Embodiments

It should be noted that in the case without conflicting, embodiments in the present disclosure and features in the embodiments may be combined with each other. The present disclosure is described in detail below in combination with the embodiments.

Term explanation:
Large sterically hindered chiral amine compound: in the present disclosure, it refers to a ketone compound with a group next to a latent chiral carbonyl that is greater than a methyl, herein the group greater than the methyl may be, for example, an ethyl, a propyl, a tert-butyl, or a phenyl tert-butyl.

In prior art, a transaminase with catalytic activity for the large sterically hindered chiral amine compound has not reported yet. In order to improve this situation, the inventor screened transaminases reported currently in large quantities and screened thousands of wild-type transaminases. Results are similar to expectations, most of the transaminases do not have the catalytic activity (namely a target product may not be detected at all). However, some transaminases (Tables 10 and 12) that may catalyze the synthesis of such large sterically hindered chiral amine compounds were still found. Although the activity (the conversion rate of catalyzing a substrate) is relatively low (less than 1%), it at least provides a basis for potential improvement.

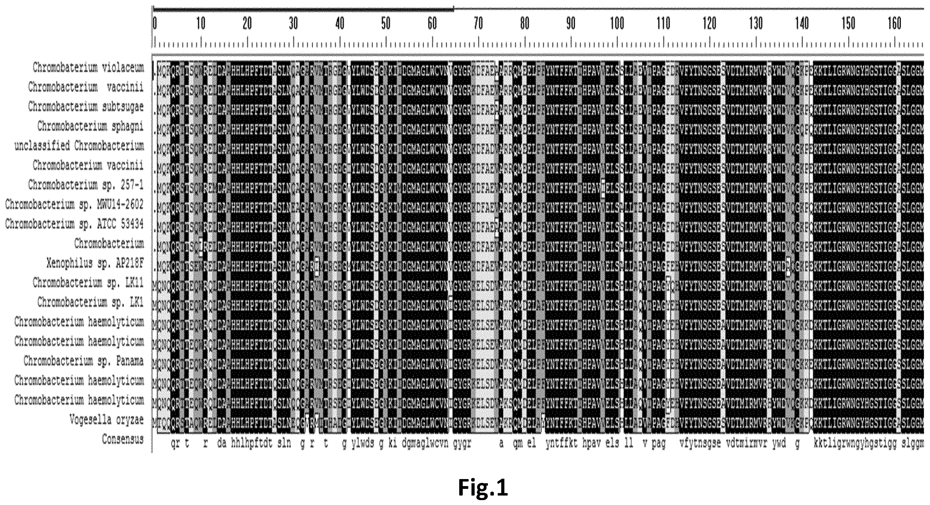

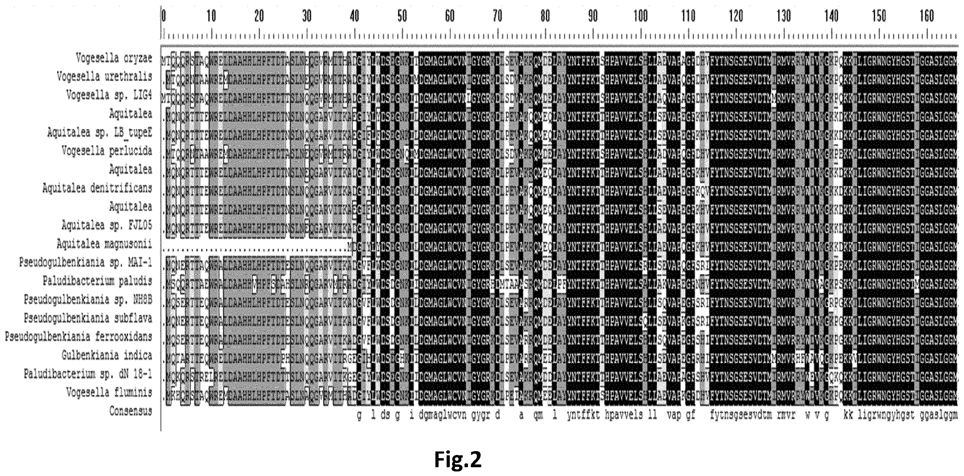

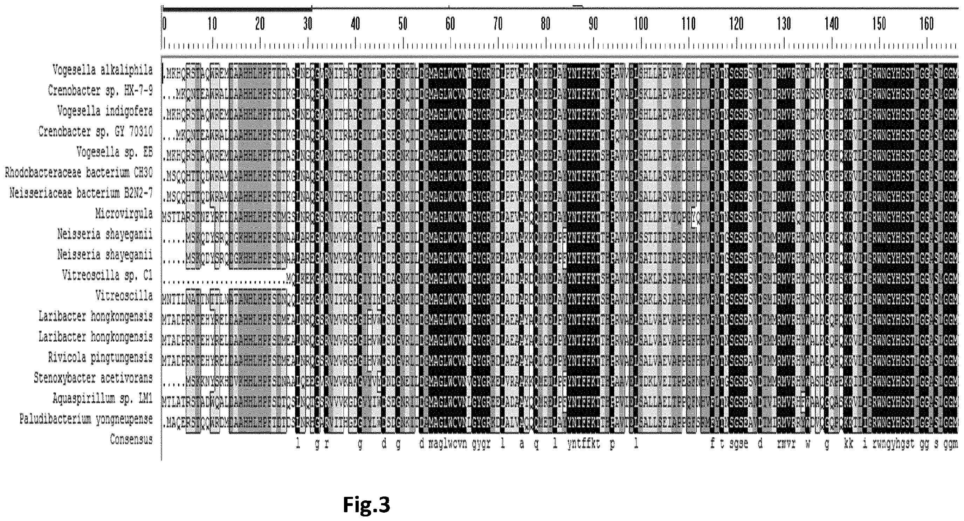

The inventor compares these transaminases of the same genus or different genera with certain catalytic activity and a CvTA transaminase of Chromobaterium violaceum (the wild-type sequence is SEQ ID NO: 1), and uses the CvTA transaminase as a reference sequence for amino acid sequence alignment. Alignment results are shown in Figs 1 to 3, and it was discovered that there are many conserved amino acid sequence regions. For example, T in the 37-th site, G in the 41-th site, YLWDS in the 43-37-th sites, G in the 49-th site, KI in the 51-52-th sites, DGMAGLWCVN in the 54-63-th sites, GYGR in the 66-69-th sites, A in the 75-th site, QM in the 78-79-th sites, EL in the 81-82-th sites, and FYNTFFKTTHPAV in the 84-96-th sites.

It may be seen from the results of multi-sequence alignment that two regions, the conserved amino acid sequence region from the 54-th site to the 63-th site and the conserved amino acid sequence region from the 84-th site to the 96-th site, are highly conserved in the transaminase of Chromobacterium genus, and both are structurally located near the substrate binding site, which are relatively important amino acids. In general, mutations at highly conserved sites in the protein sequence are easy to cause loss of the enzyme activity, so the mutations at the highly conserved sites are generally not selected. Such transaminases have certain transaminase activity, and it may be helpful to improve the catalytic activity of such transaminases on the large sterically hindered substrate after some mutations of the amino acids in such conserved amino acid sequence regions.

In order to further verify that the mutations of these conserved amino acid sites improve the catalytic activity of such large sterically hindered substrates, the inventor firstly tested the CvTA transaminase derived from *Chromobaterium violaceum* (the wild-type sequence is SEQ ID NO: 1), and a variant of the CvTA transaminase with the following mutation as an evolutionary female parent: T7C, S47C, F89Y, M166F, Y168A, V379A, Q380L, R416D and A417S, and the amino acid sequence of the female parent is recorded as SEQ ID NO: 2.

The sequence of SEQ ID NO: 1 is as follows (459 amino acids):

MQKQRTTSQWRELDAAHHLHPFTDTASLNQAGARVMTRGEGVYLWDSEGNKIIDGMAGLW
CVNVGYGRKDFAEAARRQMEELPFYNTFFKTTHPAVVELSSLLAEVTPAGFDRVFYTNSGSESVD
TMIRMVRRYWDVQGKPEKKTLIGRWNGYHGSTIGGASLGGMKYMHEQGDLPIPGMAHIEQPWW
YKHGKDMTPDEFGVVAARWLEEKILEIGADKVAAFVGEPIQGAGGVIVPPATYWPEIERICRKYDVL
LVADEVICGFGRTGEWFGHQHFGFQPDLFTAAKGLSSGYLPIGAVFVGKRVAEGLIAGGDFNHGFT
YSGHPVCAAVAHANVAALRDEGIVQRVKDDIGPYMQKRWRETFSRFEHVDDVRGVGMVQAFTLV

KNKAKRELFPDFGEIGTLCRDIFFRNNLIMRACGDHIVSAPPLVMTRAEVDEMLAVAERCLEEFEQT
LKARGLA.

The sequence of SEQ ID NO: 2 is as follows.

MQKQRTCSQWRELDAAHHLHPFTDTASLNQAGARVMTRGEGVYLWDCEGNKIIDGMAGLW
CVNVGYGRKDFAEAARRQMEELPFYNTFYKTTHPAVVELSSLLAEVTPAGFDRVFYTNSGSESVD
TMIRMVRRYWDVQGKPEKKTLIGRWNGYHGSTIGGASLGGFKAMHEQGDLPIPGMAHIEQPWW
YKHGKDMTPDEFGVVAARWLEEKILEIGADKVAAFVGEPIQGAGGVIVPPATYWPEIERICRKYDVL
LVADEVICGFGRTGEWFGHQHFGFQPDLFTAAKGLSSGYLPIGAVFVGKRVAEGLIAGGDFNHGFT
YSGHPVCAAVAHANVAALRDEGIVQRVKDDIGPYMQKRWRETFSRFEHVDDVRGVGMALAFTLV
KNKAKRELFPDFGEIGTLCRDIFFRNNLIMDSCGDHIVSAPPLVMTRAEVDEMLAVAERCLEEFEQT
LKARGLA.

Further, a series of transaminase mutants were obtained by protein engineering on the basis of the female parent, and the results showed that the catalytic efficiency of these transaminase mutants on a variety of ketone substrates was greatly improved, and they may be used for the synthesis of a variety of chiral amine compounds, especially for the efficient synthesis of the large sterically hindered chiral amine compounds.

Furthermore, the inventor performed the same mutation at the same conserved site for the wild-type transaminases derived from different sources mentioned above (i.e. SEQ ID NO: 3 to SEQ ID NO: 56). For example, according to the mutation of L59A on the basis of SEQ ID NO: 1, the same mutation of L to A occurs on the corresponding conserved site of the transaminase from other species sources, was discovered to have significantly improved catalytic activity on the large sterically hindered substrates. Similarly, according to the mutation of L59A+F88A occurring on the basis of SEQ ID NO: 1, the same mutation on the corresponding conserved amino acid site of the transaminase from other species also obtained the catalytic activity on the large sterically hindered substrates.

In the part of embodiment, the conserved amino acid mutations at positions 54-63 and 84-96 of SEQ ID NO: 1 will be taken as examples to illustrate in detail the change of catalytic activity of wild type transaminase from different species on large steric substrate after the same amino acid mutation in the same conserved amino acid site region as CvTA transaminase.

Based on this, in a preferred embodiment of the present disclosure, the above transaminase mutant is selected from:
(1) a mutant obtained from a mutation of amino acids of SEQ ID NO: 2, wherein the mutation of amino acids is any one of the group consisting of: F22A, L59A, W60A, C61A, F88A, Y89A, K90A, T107A, N151A, Y153A, F166A, E168A, E171A, K193A, V234A, I262A, I297A, K304A, F320A, T321A, E368A, L379A, L380A, F397A, R405A, F409A, D416A, S417A, C418A, and S424A; or
(2) a mutant obtained from a mutation of amino acids of SEQ ID NO: 2, wherein the mutation of amino acids is F88A and any one or more of mutations in the following positions: L59, P83, F84, Y85, N86, T87, Y89, K90, T91, F409, and A417; or
(3) a mutant obtained from a mutation of conserved amino acids of a wild-type transaminase, wherein the conserved amino acids are located at positions 54 to 63 and positions 84 to 96 of the SEQ ID NO: 1 with the sequence SEQ ID NO: 1 of the wild-type CvTA transaminase as a reference, and the conserved amino acid undergoes at least any one of the following mutations: L59A, W60A, C61A, F84S/C/G/L/R/V/P/A/M/E/K/W/Q/T/H/D/Y/N/I, Y85M, N86H/Q/P/D/K/Y/S/L/M/I/T/W/F/V/R/S/K/G, T87K/C/E/N/Q/H/F/R/D/I/M/W/A/P/V/S/L/G, F88A, Y89D/A/, K90A/F/G/D/C/S/A/E/L/V/I/R/T/Y/M/H/N/P/Q/W, and T91M/W/V/G/C/R/A/F/I/M/E/L/S/K/H/Q/D/N/P/Y, wherein "/" represents "or".

In a preferred embodiment of the present disclosure, the above wild-type transaminase is a wild-type transaminase having more than 69%, preferably more than 75%, more preferably more than 80%, and further preferably more than 85% of identity with SEQ ID NO: 1, and having the conserved amino acid. For example, it may be a wild-type transaminase with 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or more than 99% of the identity. For example, it may also be a wild-type transaminase with 99.1%, 99.2%, 99.3%, 99.4%, 99.5%, 99.6%, 99.7%, 99.8% or even more than 99.9% of the identity.

In a preferred embodiment of the present disclosure, the wild-type transaminase is derived from any one of the 54 sources indicated by the enzyme numbers 1 to 54.

In a preferred embodiment of the present disclosure, the conserved amino acid undergoes any one of the following combined mutations: (a) L59A+F88A; (b) L59A+F88A+F89D; (c) L59A+F88A+F89D+N86H; and (d) L59A+F88A+F89D+N86H+Y85M.

In a preferred embodiment of the present disclosure, the transaminase mutant is a transaminase mutant shown in Tables 1 to 9.

The catalytic activity of the above transaminase mutants on the ketone substrate is significantly improved compared with the wild type, and the synthesis of the large sterically hindered chiral amine compounds catalyzed by the transaminase has been realized, the green chemical synthesis is achieved without the use of heavy metal catalysts. In addition, a series of chiral amine compounds with high activity and high selectivity may be easily obtained with the transaminase having increased activity for the transamination reaction.

In a typical embodiment of the present disclosure, a DNA molecule is further provided, and the DNA molecule encodes any one of the transaminase mutants mentioned above. The transaminase mutant encoded by the DNA molecule has the higher catalytic activity for a series of the ketone substrate compounds, and it is beneficial to the synthesis of such chiral amine compounds by a method of biological enzyme catalysis.

The above DNA molecule of the present disclosure may also be existed in the form of an "expression cassette". The "expression cassette" refers to a linear or a circular nucleic acid molecule that encompasses DNA and RNA sequences capable of guiding the expression of a specific nucleotide sequence in an appropriate host cell. In general, it includes a promoter that is operably linked to a target nucleotide, and it is optionally operably linked to a termination signal and/or other regulatory elements. The expression cassette may also include a sequence required for correct translation of the nucleotide sequence. The encoding region usually encodes a target protein, but also encodes a target functional RNA in the sense or antisense direction, such as an antisense RNA or an untranslated RNA. The expression cassette containing the target polynucleotide sequence may be chimeric, it means that at least one of its components is heterologous to at least one of its other components. The expression cassette may also be naturally present, but obtained by effective recombination for heterologous expression.

In a typical embodiment of the present disclosure, a recombinant plasmid is further provided, and the recombinant plasmid is linked with the aforementioned DNA molecule. The DNA molecule in the above recombinant plasmid is placed in an appropriate position of the recombinant plasmid, so that the above DNA molecule may be correctly and smoothly replicated, transcribed or expressed.

Although the present disclosure uses a qualifier "containing" while the above DNA molecule is defined, it does not mean that other sequences unrelated to its functions may be arbitrarily added at both ends of the DNA sequence. Those skilled in the art are aware that in order to meet the requirements of recombination operations, appropriate restriction endonuclease cleavage sites need to be added at both ends of the DNA sequence, or a start codon, a stop codon and the like are additionally added. Therefore, the use of closed-type statement for definition may not truly cover these situations.

The term "plasmid" used in the present disclosure includes any plasmids, cosmids, bacteriophages, or agrobacterium binary nucleic acid molecules in the double or single-stranded linear or circular form, preferably a recombinant expression plasmid, and it may be a prokaryotic expression plasmid or an eukaryotic expression plasmid, but preferably the prokaryotic expression plasmid. In some implementation schemes, the recombinant plasmid is selected from pET-21b(+), pET-22b(+), pET-3a(+), pET-3d(+), pET-11a(+), pET-12a(+), pET-14b, pET-15b(+), pET-16b(+), pET-17b(+), pET-19b(+), pET-20b(+), pET-21a(+), pET-23a(+), pET-23b(+), pET-24a(+), pET-25b(+), pET-26b(+), pET-27b(+), pET-28a(+), pET-29a(+), pET-30a(+), pET-31b(+), pET-32a(+), pET-35b(+), pET-38b(+), pET-39b(+), pET-40b(+), pET-41a (+), pET-41b(+), pET-42a(+), pET-43a(+), pET-43b(+), pET-44a(+), pET-49b(+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-8, pUC-18 or pUC-19. More preferably, the above recombinant plasmid is pET-22b(+).

According to a typical implementation scheme of the present disclosure, a host cell is provided, and the host cell contains any one of the above recombinant plasmids. The host cell applicable to the present disclosure includes but not limited to a prokaryotic cell or a eukaryotic cell. Preferably, the prokaryotic cell is an Escherichia coli BL21 cell or an Escherichia coli DH5α competent cell, and the eukaryotic cell is yeast.

In a typical embodiment of the present disclosure, a synthesis method for a chiral amine compound is further provided, and the synthesis method uses any one of the above transaminase mutants to transaminate a ketone substrate shown in Formula I under the action of an amino donor, to obtain the chiral amine compound; 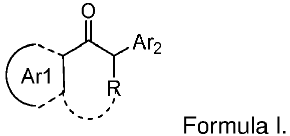 herein Ar1 represents a substituted or unsubstituted aryl, a substituted or unsubstituted arylene, or a substituted or unsubstituted heteroarylene; Ar2 represents a substituted or unsubstituted aryl, or a substituted or unsubstituted cycloalkyl; and optionally R represents a substituted or unsubstituted C1-C5 alkylene, and the R is connected to Ar1 or Ar2 to form a ring; wherein a substituent in the substituted aryl, the substituted aryl arylene, the substituted heteroarylene or the substituted alkylene is independently selected from halogen, hydroxy, or amino, and a heteroatom in the substituted heteroarylene is selected from N, O or S.

Since the above transaminase of the present disclosure has the higher enzyme catalytic activity for the substrate shown in Formula I, the preparation of the chiral amine compound, especially the large sterically hindered chiral amine compound, with the transaminase mutant of the present disclosure may not only reduce the production cost, but also obtain the higher purity and enantiomeric excess (ee) value of the large sterically hindered chiral amine.

The substituent in the above substituted aryl or arylene is halogen or -S-CH₃, the halogen or -S-CH₃ is located at any one or more positions in an ortho-position, a meta-position, or a para-position of the aryl or arylene, and preferably, the halogen is F, Cl, or Br.

In a preferred embodiment, the Ar1 represents a substituted or unsubstituted aryl, or a substituted or unsubstituted arylene, the Ar2 represents an unsubstituted aryl, and the R represents an unsubstituted C1~C5 alkylene, and the R is connected to Ar1 to form a ring.

In a preferred embodiment, the Ar1 represents a unsubstituted heteroarylene, the Ar2 represents a halogen-substituted aryl, and the R represents a hydroxyl-substituted C1~C5 alkylene, and the R is connected to Ar1 to form a ring.

In a preferred embodiment, the Ar1 represents a halogen- and amino-substituted arylene, and the Ar2 represents a C3-C8 cycloalkyl.

In a preferred embodiment, the Ar1 represents an unsubstituted cycloalkyl or an unsubstituted aryl, and the Ar2 represents a substituted or unsubstituted cycloalkyl, or a substituted or unsubstituted aryl.

In a preferred embodiment, the Ar1 represents a hydroxyl-, a methyl-, an ethyl-, or a -S-CH3-substituted cycloalkyl, or a hydroxyl-, a methyl-, an ethyl-, or a -S-CH3-substituted aryl, and the Ar2 represents an unsubstituted aryl or an unsubstituted alky.

In a preferred embodiment, the ketone substrate is:

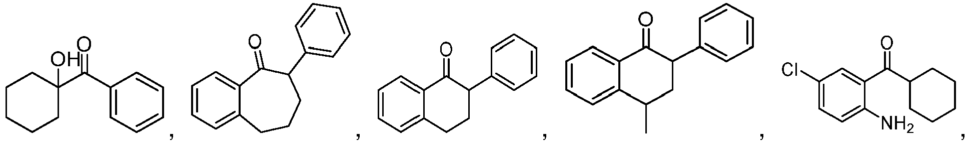

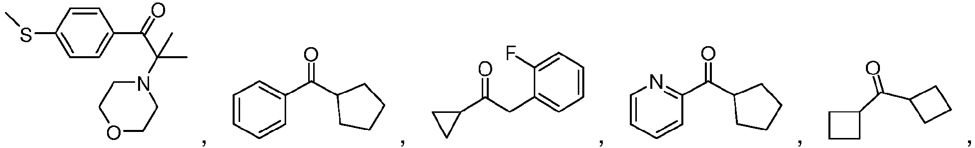

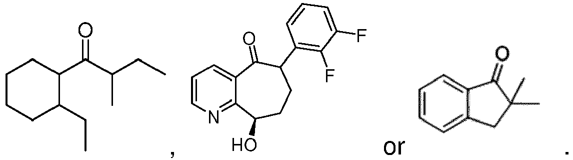

In a preferred embodiment, the amino donor is isopropylamine, isopropylamine hydrochloride, alanine, n-butylamine, or aniline.

The above technical schemes and technical effects are described below in combination with specific embodiments. The substrates used in the following embodiments are as follows.

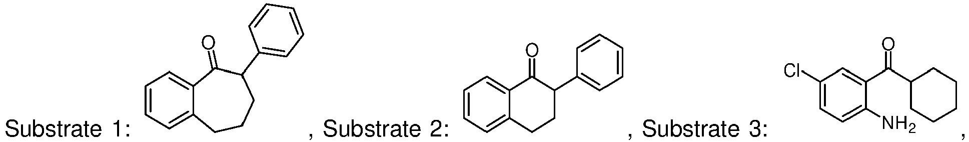

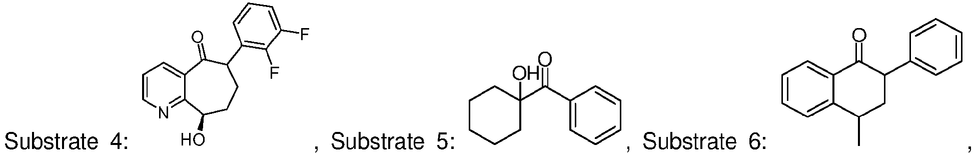

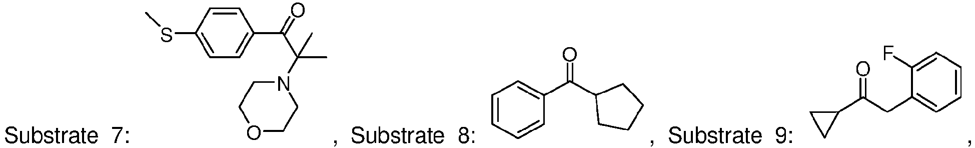

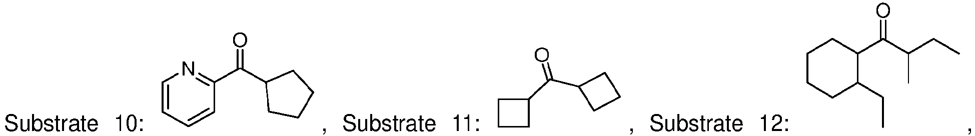

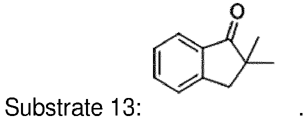

### (I) Part one: SEQ ID NO: 2 is used as a female parent for modification.

### Embodiment 1

A directed mutation was performed on the basis of the female parent, specific mutation sites were shown in the table below, and the catalytic activity of the mutant was detected according to the following reaction conditions:
1 mL of a system including 1 mg of Substrate 1, 1 mg of pyridoxal phosphate (PLP), 1 mg of isopropylamine hydrochloride, 50 mg of enzyme powder, and 100 mM of pH 8.0 phosphate buffer, and the reaction was performed at 50°C for 40 h.

Detection results were shown in the table below:

**Table 1:**

| Mutant | Activity (%) |
|---|---|
| Female parent | 0 |
| F22A | + |
| L59A | ++ |
| W60A | ++ |
| C61A | + |
| F88A | ++ |
| Y89A | + |
| K90A | + |
| T107A | + |
| N151A | + |
| Y153A | + |
| F166A | + |
| E171A | + |
| K193A | + |
| V234A | + |
| I262A | + |
| I297A | + |
| K304A | + |
| F320A | + |
| T321A | + |
| E368A | + |
| L380A | + |
| F397A | + |
| R405A | + |
| F409A | + |
| D416A | + |
| S417A | + |
| C418A | + |
| S424A | + |
| F166A | + |
| E171A | + |

| | |
|---|---|
| Note: in the above table, 0 represented the conversion rate was less than 1%, + represented the conversion rate was greater than or equal to 1% and less than 5%, and ++ represented the conversion rate was greater than or equal to 5% and less than or equal to 10%. | |

### Embodiment 2

Based on Embodiment 1, a combined mutation was performed, and activity screening was performed on the combined mutation under the same reaction conditions as Embodiment 1. Results were shown in the table below.

**Table 2:**

| Mutant | Activity(%) |
|---|---|
| F88A | ++ |
| F88A+L59H | +++ |
| F88A+L59R | +++ |
| F88A+L59F | +++ |
| F88A+L59Y | +++ |
| F88A+L59S | +++ |
| F88A+L59C | +++ |
| F88A+L59Q | +++ |
| F88A+L59M | +++ |
| F88A+L59L | +++ |
| F88A+L59V | +++ |
| F88A+L59G | +++ |
| F88A+L59A | ++++ |
| F88A+L59D | +++ |
| F88A+L59N | +++ |
| F88A+L59R | +++ |
| F88A+L59W | +++ |
| F88A+L59P | +++ |
| F88A+L59I | +++ |

| | |
|---|---|
| Note: in the above table, 0 represented the conversion rate was less than 1%, + represented the conversion rate was greater than or equal to 1% and less than 5%, ++ represented the conversion rate was greater than or equal to 5% and less than 10%, +++ represented the conversion rate was greater than or equal to 10% and less than 15%, and ++++ represented the conversion rate was greater than or equal to 15% and less than 20%. | |

### Embodiment 3

Based on Embodiment 2, a combined mutation was performed, and activity screening was performed on the combined mutation under the same reaction conditions as Embodiment 1. Results were shown in the table below.

**Table 3:**

| Mutant | Activity (%) |
|---|---|
| F88A+L59A | ++++ |
| F88A+L59A+Y89G | ++++ |
| F88A+L59A+Y89L | ++++ |
| F88A+L59A+Y89S | ++++ |
| F88A+L59A+Y89V | ++++ |
| F88A+L59A+Y89P | ++++ |
| F88A+L59A+Y89A | ++++ |
| F88A+L59A+Y89W | ++++ |
| F88A+L59A+Y89M | ++++ |
| F88A+L59A+Y89I | ++++ |
| F88A+L59A+Y89D | +++++ |
| F88A+L59A+Y89R | ++++ |
| F88A+L59A+Y89F | ++++ |
| F88A+L59A+Y89H | ++++ |
| F88A+L59A+Y89Q | ++++ |
| F88A+L59A+Y89N | ++++ |
| F88A+L59A+Y89E | ++++ |
| F88A+L59A+Y89C | ++++ |
| F88A+L59A+Y89T | ++++ |
| F88A+L59A+Y89K | ++++ |

| | |
|---|---|
| Note: 0 represented the conversion rate was less than 1%, + represented the conversion rate was greater than or equal to 1% and less than 5%, ++ represented the conversion rate was greater than or equal to 5% and less than 10%, +++ represented the conversion rate was greater than or equal to 10% and less than 15%, ++++ represented the conversion rate was greater than or equal to 15% and less than 20%, and +++++ represented the conversion rate was greater than or equal to 20% and less than or equal to 40%. | |

### Embodiment 4

Based on Embodiment 3, a combined mutation was performed, and activity screening was performed on the combined mutation under the same reaction conditions as Embodiment 1. Results were shown in the table below.

**Table 4:**

| Mutant | Activity (%) |
|---|---|
| F88A+L59A+Y89D | +++++ |
| F88A+L59A+Y89D+T87G | +++++ |
| F88A+L59A+Y89D+T87L | +++++ |
| F88A+L59A+Y89D+T87S | +++++ |
| F88A+L59A+Y89D+T87V | +++++ |
| F88A+L59A+Y89D+T87P | +++++ |
| F88A+L59A+Y89D+T87A | +++++ |
| F88A+L59A+Y89D+T87W | +++++ |
| F88A+L59A+Y89D+T87M | +++++ |
| F88A+L59A+Y89D+T87I | +++++ |
| F88A+L59A+Y89D+T87D | +++++ |
| F88A+L59A+Y89D+T87R | +++++ |
| F88A+L59A+Y89D+T87F | +++++ |
| F88A+L59A+Y89D+T87H | +++++ |
| F88A+L59A+Y89D+T87Q | +++++ |
| F88A+L59A+Y89D+T87N | +++++ |
| F88A+L59A+Y89D+T87E | +++++ |
| F88A+L59A+Y89D+T87C | +++++ |
| F88A+L59A+Y89D+T87K | +++++ |
| F88A+L59A+Y89D+N86G | +++++ |
| F88A+L59A+Y89D+N86C | +++++ |
| F88A+L59A+Y89D+N86S | +++++ |
| F88A+L59A+Y89D+N86R | ++++ |
| F88A+L59A+Y89D+N86V | +++++ |
| F88A+L59A+Y89D+N86F | +++++ |
| F88A+L59A+Y89D+N86W | +++++ |
| F88A+L59A+Y89D+N86T | +++++ |
| F88A+L59A+Y89D+N86I | +++++ |
| F88A+L59A+Y89D+N86M | ++++++ |
| F88A+L59A+Y89D+N86L | +++++ |
| F88A+L59A+Y89D+N86A | +++++ |
| F88A+L59A+Y89D+N86Y | +++++ |
| F88A+L59A+Y89D+N86K | +++++ |
| F88A+L59A+Y89D+N86D | +++++ |
| F88A+L59A+Y89D+N86Q | ++++++ |
| F88A+L59A+Y89D+N86P | +++++ |
| F88A+L59A+Y89D+N86H | ++++++ |
| F88A+L59A+Y89D+N86E | +++++ |

| | |
|---|---|
| Note: in the above table, 0 represented the conversion rate was less than 1%, + represented the conversion rate was greater than or equal to 1% and less than 5%, ++ represented the conversion rate was greater than or equal to 5% and less than 10%, +++ represented the conversion rate was greater than or equal to 10% and less than 15%, ++++ represented the conversion rate was greater than or equal to 15% and less than 20%, +++++ represented the conversion rate was greater than or equal to 20% and less than 40%, and ++++++ represented the conversion rate was greater than or equal to 40%. | |

### Embodiment 5

Based on Embodiment 4, a combined mutation was performed, specific mutation sites were shown in the table below, and the catalytic activity of the mutant was detected according to the following reaction conditions:

1 mL of a system included 10 mg of Substrate 1, 1 mg of PLP, 1 mg of isopropylamine hydrochloride, 5 mg of enzyme powder, and 100 mM of pH 8.0 phosphate buffer, it was reacted at 50°C for 40 h, and results were shown in the table below.

**Table 5:**

| Mutant | Activity (%) |
|---|---|
| F88A+L59A+Y89D+N86H | + |
| F88A+L59A+Y89D+N86H+F84S | + |
| F88A+L59A+Y89D+N86H+F84C | + |
| F88A+L59A+Y89D+N86H+F84G | + |
| F88A+L59A+Y89D+N86H+F84L | + |
| F88A+L59A+Y89D+N86H+F84R | + |
| F88A+L59A+Y89D+N86H+F84V | + |
| F88A+L59A+Y89D+N86H+F84P | + |
| F88A+L59A+Y89D+N86H+F84A | + |
| F88A+L59A+Y89D+N86H+F84M | + |
| F88A+L59A+Y89D+N86H+F84E | + |
| F88A+L59A+Y89D+N86H+F84K | + |
| F88A+L59A+Y89D+N86H+F84W | + |
| F88A+L59A+Y89D+N86H+F84Q | + |
| F88A+L59A+Y89D+N86H+F84T | + |
| F88A+L59A+Y89D+N86H+F84H | + |
| F88A+L59A+Y89D+N86H+F84D | + |
| F88A+L59A+Y89D+N86H+F84Y | + |
| F88A+L59A+Y89D+N86H+F84N | + |
| F88A+L59A+Y89D+N86H+F84I | + |
| F88A+L59A+Y89D+N86H+Y85K | + |
| F88A+L59A+Y89D+N86H+Y85C | ++ |
| F88A+L59A+Y89D+N86H+Y85R | ++ |
| F88A+L59A+Y89D+N86H+Y85M | ++ |
| F88A+L59 A+Y89D+N86H+Y85I | ++ |
| F88A+L59A+Y89D+N86H+Y85L | + |
| F88A+L59A+Y89D+N86H+Y85Q | + |
| F88A+L59A+Y89D+N86H+Y85S | + |
| F88A+L59A+Y89D+N86H+Y85P | + |
| F88A+L59A+Y89D+N86H+Y85D | + |
| F88A+L59A+Y89D+N86H+Y85V | + |
| F88A+L59A+Y89D+N86H+Y85A | + |
| F88A+L59A+Y89D+N86H+Y85H | + |
| F88A+L59A+Y89D+N86H+Y85W | + |
| F88A+L59A+Y89D+N86H+Y85T | + |
| F88A+L59A+Y89D+N86H+Y85F | + |
| F88A+L59A+Y89D+N86H+Y85E | + |
| F88A+L59A+Y89D+N86H+Y85N | + |
| F88A+L59A+Y89D+N86H+Y85G | + |
| F88A+L59A+Y89D+N86H+Y85A | + |

| | |
|---|---|
| Note: in the above table, + represented the conversion rate was greater than or equal to 0% and less than 20%, and ++ represented the conversion rate was greater than or equal to 20% and less than 40%. | |

### Embodiment 6

Based on Embodiment 5, a combined mutation was performed, and activity screening was performed on the combined mutation under the same reaction conditions as Embodiment 5. Results were shown in the table below.

**Table 6:**

| Mutant | Activity (%) |
|---|---|
| F88A+L59A+Y89D+N86H+Y85M | ++ |
| F88A+L59A+Y89D+N86H+Y85M+K90F | ++ |
| F88A+L59A+Y89D+N86H+Y85M+K90G | +++ |
| F88A+L59A+Y89D+N86H+Y85M+K90D | ++ |
| F88A+L59A+Y89D+N86H+Y85M+K90C | ++ |
| F88A+L59A+Y89D+N86H+Y85MK90S | ++ |
| F88A+L59A+Y89D+N86H+Y85M+K90A | ++ |
| F88A+L59A+Y89D+N86H+Y85M+K90E | ++ |
| F88A+L59A+Y89D+N86H+Y85M+K90L | ++ |
| F88A+L59A+Y89D+N86H+Y85M+K90V | ++ |
| F88A+L59A+Y89D+N86H+Y85M+K90I | ++ |
| F88A+L59A+Y89D+N86H+Y85M+K90R | ++ |
| F88A+L59A+Y89D+N86H+Y85M+K90T | ++ |
| F88A+L59A+Y89D+N86H+Y85M+K90Y | ++ |
| F88A+L59A+Y89D+N86H+Y85M+K90M | ++ |
| F88A+L59A+Y89D+N86H+Y85M+K90H | ++ |
| F88A+L59A+Y89D+N86H+Y85M+K90N | ++ |
| F88A+L59A+Y89D+N86H+Y85M+K90P | ++ |
| F88A+L59A+Y89D+N86H+Y85M+K90Q | ++ |
| F88A+L59A+Y89D+N86H+Y85M+K90W | ++ |
| F88A+L59A+Y89D+N86HY85M+Y91W | ++ |
| F88A+L59A+Y89 D+N86H+Y85M+T91V | ++ |
| F88A+L59A+Y89D+N86H+Y85M+T91G | ++ |
| F88A+L59A+Y89D+N86H+Y85M+T91C | ++ |
| F88A+L59A+Y89D+N86H+Y85M+T91R | ++ |
| F88A+L59A+Y89D+N86H+Y85M+T91A | ++ |
| F88A+L59A+Y89D+N86H+Y85M+T91F | ++ |
| F88A+L59A+Y89D+N86H+Y85M+T91I | +++ |
| F88A+L59A+Y89D+N86H+Y85M+T91M | ++ |
| F88A+L59A+Y89D+N86H+Y85M+T91E | ++ |
| F88A+L59A+Y89D+N86H+Y85M+T91L | ++ |
| F88A+L59A+Y89D+N86H+Y85M+T91S | ++ |
| F88A+L59A+Y89D+N86H+Y85M+T91K | ++ |
| F88A+L59A+Y89D+N86H+Y85M+T91H | ++ |
| F88A+L59A+Y89D+N86H+Y85M+T91Q | ++ |
| F88A+L59A+Y89D+N86H+Y85M+T91D | ++ |
| F88A+L59A+Y89D+N86H+Y85M+T91N | +++ |
| F88A+L59A+Y89D+N86H+Y85M+T91P | ++ |
| F88A+L59A+Y89D+N86H+Y85M+T91Y | ++ |

| | |
|---|---|
| Note: + represented the conversion rate was greater than or equal to 0% and less than 20%, ++ represented the conversion rate was greater than or equal to 20% and less than 40%, and +++ represented the conversion rate was greater than or equal to 40% and less than 80%. | |

### Embodiment 7

Based on Embodiment 6, a combined mutation was performed, and activity screening was performed on the combined mutation under the same reaction conditions as Embodiment 5. Results were shown in the table below.

**Table 7:**

| Mutant | Activity (%) |
|---|---|
| F88A+L59A+Y89D+N86H+Y85M+K90G+T91M | +++ |
| F88A+L59A+Y89D+N86H+Y85M+K90G+T91M+P83F | +++ |
| F88A+L59A+Y89D+N86H+Y85M+K90G+T91M+P83G | +++ |
| F88A+L59A+Y89D+N86H+Y85M+K90G+T91M+P83D | +++ |
| F88A+L59A+Y89D+N86H+Y85M+K90G+T91M+P83C | ++++ |
| F88A+L59A+Y89D+N86H+Y85M+K90G+T91M+P83S | ++++ |
| F88A+L59A+Y89D+N86H+Y85N+K90G+T91M+P83A | +++ |
| F88A+L59A+Y89D+N86H+Y85M+K90G+T91M+P83E | +++ |
| F88A+L59A+Y89D+N86H+Y85M+K90G+T91M+P83L | +++ |
| F88A+L59A+Y89D+N86H+Y85M+K90G+T91M+P83V | +++ |
| F88A+L59A+Y89D+N86H+Y85N+K90G+T91M+P83I | +++ |
| F88A+L59A+Y89D+N86H+Y85M+K90G+T91M+P83R | +++ |
| F88A+L59A+Y89D+N86H+Y85M+K90G+T91M+P83T | +++ |
| F88A+L59A+Y89D+N86H+Y85M+K90G+T91M+P83Y | +++ |
| F88A+L59A+Y89D+N86H+Y85M+K90G+T91M+P83M | +++ |
| F88A+L59A+Y89D+N86H+Y85M+K90G+T91M+P83H | +++ |
| F88A+L59A+Y89D+N86H+Y85M+K90G+T91M+P83N | +++ |
| F88A+L59A+Y89D+N86H+Y85M+K90G+T91M+P83K | +++ |
| F88A+L59A+Y89D+N86H+Y85M+K90G+T91M+P83Q | +++ |
| F88A+L59A+Y89D+N86H+Y85M+K90G+T91M+P83W | +++ |
| F88A+L59A+Y89D+N86H+Y85M+K90G+T91M+F409Y | ++++ |
| F88A+L59A+Y89D+N86H+Y85M+K90G+T91M+F409I | +++ |
| F88A+L59A+Y89D+N86H+Y85M+K90G+T91M+F409G | +++ |
| F88A+L59A+Y89D+N86H+Y85M+K90G+T91M+F409F | +++ |
| F88A+L59A+Y89D+N86H+Y85M+K90G+T91M+F409A | +++ |
| F88A+L59A+Y89D+N86H+Y85N+K90G+T91M+F409L | +++ |
| F88A+L59A+Y89D+N86H+Y85M+K90G+T91M+F409W | +++ |
| F88A+L59A+Y89D+N86H+Y85M+K90G+T91M+F409N | +++ |
| F88A+L59A+Y89D+N86H+Y85M+K90G+T91M+S417F | ++++ |
| F88A+L59A+Y89D+N86H+Y85M+K90G+T91M+S417E | +++ |
| F88A+L59A+Y89D+N86H+Y85M+K90G+T91M+S417Y | +++ |
| F88A+L59A+Y89D+N86H+Y85M+K90G+T91M+S417M | +++ |
| F88A+L59A+Y89D+N86H+Y85M+K90G+T91M+S417A | +++ |
| F88A+L59A+Y89D+N86H+Y85M+K90G+T91M+S417Q | ++++ |
| F88A+L59A+Y89D+N86H+Y85M+K90G+T91M+S417C | +++ |
| F88A+L59A+Y89D+N86H+Y85M+K90G+T91M+S417L | +++ |
| F88A+L59A+Y89D+N86H+Y85M+K90G+T91M+S417I | ++++ |
| F88A+L59A+Y89D+N86H+Y85M+K90G+T91M+S417T | +++ |
| F88A+L59A+Y89D+N86H+Y85M+K90G+T91M+S417V | +++ |
| F88A+L59A+Y89D+N86H+Y85M+K90G+T91M+S417W | +++ |
| F88A+L59A+Y89D+N86H+Y85M+K90G+T91M+S424N | +++ |
| F88A+L59A+Y89D+N86H+Y85M+K90G+T91M+S424F | +++ |
| F88A+L59A+Y89D+N86H+Y85M+K90G+T91M+S424T | +++ |
| F88A+L59A+Y89D+N86H+Y85M+K90G+T91M+S424G | +++ |
| F88A+L59A+Y89D+N86H+Y85M+K90G+T91M+S424I | +++ |
| F88A+L59A+Y89D+N86H+Y85M+K90G+T91M+S424V | +++ |
| F88A+L59A+Y89D+N86H+Y85M+K90G+T91M+S424A | ++++ |
| F88A+L59A+Y89D+N86H+Y85M+K90G+T91M+S424L | +++ |
| F88A+L59A+Y89D+N86H+Y85M+K90G+T91M+V234A | ++++ |
| F88A+L59A+Y89D+N86H+Y85M+K90G+T91M+L380A | ++++ |
| F88A+L59A+Y89D+N86H+Y85M+K90G+T91M+C61A | +++ |

| | |
|---|---|
| Note: in the above table, + represented the conversion rate was greater than or equal to 0% and less than 20%, ++ represented the conversion rate was greater than or equal to 20% and less than 40%, +++ represented the conversion rate was greater than or equal to 40% and less than 80%, and ++++ represented the conversion rate was greater than or equal to 80%. | |

### Embodiment 8

Based on Embodiment 7, a combined mutation was performed, and activity screening was performed on the combined mutation under the same reaction conditions as Embodiment 5. Results were shown in the table below.

**Table 8:**

| Mutant | Activity (%) |
|---|---|
| F88A+L59A+Y89D+N86H+Y85M+K90G+T91M+P83S | ++++ |
| F88A+L59A+Y89D+N86H+Y85M+K90G+T91M+P83S+V234A | ++++ |
| F88A+L59A+Y89D+N86H+Y85M+K90G+T91M+P83S+V234A+L380A | ++++ |
| F88A+L59A+Y89D+N86H+Y85M+K90G+T91M+P83S+V234A+L380A+S417A | ++++ |
| F88A+L59A+Y89D+N86H+Y85M+K90G+T91M+P83S+V234A+L380A+S417C | ++++ |
| F88A+L59A+Y89D+N86H+Y85M+K90G+T91M+P83S+V234A+L380A+S417D | +++ |
| F88A+L59A+Y89D+N86H+Y85M+K90G+T91M+P83S+V234A+L380A+S417E | +++ |
| F88A+L59A+Y89D+N86H+Y85M+K90G+T91M+P83S+V234A+L380A+S417F | ++++ |
| F88A+L59A+Y89D+N86H+Y85M+K90G+T91M+P83S+V234A+L380A+S417G | +++ |
| F88A+L59A+Y89D+N86H+Y85M+K90G+T91M+P83S+V234A+L380A+S417H | ++++ |
| F88A+L59A+Y89D+N86H+Y85M+K90G+T91M+P83S+V234A+L380A+S417I | ++++ |
| F88A+L59A+Y89D+N86H+Y85M+K90G+T91M+P83S+V234A+L380A+S417K | +++ |
| F88A+L59A+Y89D+N86H+Y85M+K90G+T91M+P83S+V234A+L380A+S417L | ++++ |
| F88A+L59A+Y89D+N86H+Y85M+K90G+T91M+P83S+V234A+L380A+S417M | ++++ |
| F88A+L59A+Y89D+N86H+Y85M+K90G+T91M+P83S+V234A+L380A+S417N | ++++ |
| F88A+L59A+Y89D+N86H+Y85M+K90G+T91M+P83S+V234A+L380A+S417P | +++ |
| F88A+L59A+Y89D+N86H+Y85M+K90G+T91M+P83S+V234A+L380A+S417Q | ++++ |
| F88A+L59A+Y89D+N86H+Y85M+K90G+T91M+P83S+V234A+L380A+S417R | +++ |
| F88A+L59A+Y89D+N86H+Y85M+K90G+T91M+P83S+V234A+L380A+S417T | ++++ |
| F88A+L59A+Y89D+N86H+Y85M+K90G+T91M+P83S+V234A+L380A+S417V | ++++ |
| F88A+L59A+Y89D+N86H+Y85M+K90G+T91M+P83S+V234A+L380A+S417W | ++++ |
| F88A+L59A+Y89D+N86H+Y85M+K90G+T91M+P83S+V234A+L380A+S417Y | +++ |
| F88A+L59A+Y89D+N86H+Y85M+K90G+T91M+P83S+V234A+L380A+S417A+F409L | ++++ |
| F88A+L59A+Y89D+N86H+Y85M+K90G+T91M+P83S+V234A+L380A+S417A+S424A | ++++ |
| F88A+L59A+Y89D+N86H+Y85M+K90G+T91M+P83S+V234A+L380A+S417A+S424G | ++++ |
| F88A+L59A+Y89D+N86H+Y85M+K90G+T91M+P83S+V234A+L380A+S417A+K167I | ++++ |
| F88A+L59A+Y89D+N86H+Y85M+K90G+T91M+P83S+V234C+L380A+S417A | ++++ |

| | |
|---|---|
| Note: in the above table, + represented the conversion rate was greater than or equal to 0% and less than 20%, ++ represented the conversion rate was greater than or equal to 20% and less than 40%, +++ represented the conversion rate was greater than or equal to 40% and less than 80%, and ++++ represented the conversion rate was greater than or equal to 80%. | |

### Embodiment 9

Based on Embodiment 8, a combined mutation was performed. Reaction conditions were as follows.

1 mL of a system includes 10 mg of Substrate 1, 1 mg of PLP, 1 mg of isopropylamine hydrochloride, 3 mg of enzyme powder, and 100 mM of pH 8.0 phosphate buffer, it was reacted at 50°C for 40 h, and results were shown in the table below.

**Table 9:**

| Mutant | Activi ty(%) |
|---|---|
| F88A+L59A+Y89D+N86H+Y85M+K90G+T91M+P83S+V234A+L380A+S417I | +++ |
| F88A+L59A+Y89D+N86H+Y85M+K90G+T91M+P83S+V234A+L380A+S417I+A168E | ++++ |
| F88A+L59A+Y89D+N86H+Y85M+K90G+T91M+P83S+V234A+L380A+S417I+A168E+A3 79 L | ++++ |
| F88A+L59A+Y89D+N86H+Y85M+K90G+T91M+P83S+V234A+L380A+S417I+A168E+A3 79L+S424A | ++++ |
| F88A+L59A+Y89D+N86H+Y85M+K90G+T91M+P83S+V234A+L380A+S417I+A168E+A3 79L+S424G | ++++ |
| F88A+L59A+Y89D+N86H+Y85M+K90G+T91 M+P83S+V234C+L380A+S417I+A168E+A3 79L+S424A | ++++ |
| F88A+L59A+Y89D+N86H+Y85M+K90G+T91 M+P83S+V234A+L380A+S417I+A168E+A3 79L+S424A +F301S | ++++ |
| F88A+L59A+Y89D+N86H+Y85M+K90G+T91 M+P83S+V234A+L380A+S417I+A168E+A3 79L+S424A+F364C | ++++ |
| F88A+L59A+Y89D+N86H+Y85M+K90G+T91 M+P83S+V234A+L380A+S417I+A168E+A3 79L+S424A+T452A | ++++ |
| F88A+L59A+Y89D+N86H+Y85M+K90G+T91M+P83S+V234A+L380A+S417I+A168E+A3 79L+S424A+K358R+F449L | ++++ |
| F88A+L59A+Y89D+N86H+Y85M+K90G+T91 M+P83S+V234A+L380A+S417I+A168E+A3 79L+S424A+M180V | ++++ |
| F88A+L59A+Y89D+N86H+Y85M+K90G+T91 M+P83S+V234A+L380A+S417I+A168E+A3 79L+S424A+G164S | ++++ |
| F88A+L59A+Y89D+N86H+Y85M+K90G+T91 M+P83S+V234A+L380A+S417I+A168E+A3 79L+S424A+F301S+G164S | ++++ |
| F88A+L59A+Y89D+N86H+Y85M+K90G+T91 M+P83S+V234A+L380A+S417I+A168E+A3 79L+S424A+F301S+M180V | ++++ |
| F88A+L59A+Y89D+N86H+Y85M+K90G+T91 M+P83S+V234A+L380A+S417I+A168E+A3 79L+S424A+F301S+F449L | ++++ |
| F88A+L59A+Y89D+N86H+Y85M+K90G+T91 M+P83S+V234A+L380A+S417I+A168E+A3 79L+S424A+F301S+F409L | ++++ |
| F88A+L59A+Y89D+N86H+Y85M+K90G+T91 M+P83S+V234A+L380A+S417I+A168E+A3 79L+S424A+F301S+G164S+T452S | ++++ |
| F88A+L59A+Y89D+N86H+Y85M+K90G+T91 M+P83S+V234A+L380A+S417I+A168E+A3 79L+S424A+F301S+G164S+F449L | ++++ |
| F88A+L59A+Y89D+N86H+Y85M+K90G+T91M+P83S+V234A+L380A+S417I+A168E+A3 79L+S424A+F301S+G164S+M180V | ++++ |
| F88A+L59A+Y89D+N86H+Y85M+K90G+T91 M+P83S+V234A+L380A+S417I+A168E+A3 79L+S424A+F301S+G164S+M180V+E171V | ++++ |
| F88A+L59A+Y89D+N86H+Y85M+K90G+T91 M+P83S+V234A+L380A+S417I+A168E+A3 79L+S424A+F301S+G164S+F449L+L453V | ++++ |
| F88A+L59A+Y89D+N86H+Y85M+K90G+T91 M+P83S+V234A+L380A+S417I+A168E+A3 79L+S424A+F301S+G164S+K167I | ++++ |
| F88A+L59A+Y89D+N86H+Y85M+K90G+T91 M+P83S+V234A+L380A+S417I+A168E+A3 79L+S424A+F301S+G164S+T452S+M180V | ++++ |
| F88A+L59A+Y89D+N86H+Y85M+K90G+T91 M+P83S+V234A+L380A+S417I+A168E+A3 79L+S424A+F301S+G164S+T452S+F449L | ++++ |
| F88A+L59A+Y89D+N86H+Y85M+K90G+T91 M+P83S+V234A+L380A+S417I+A168E+A3 79L+S424A+F301S+G164S+T452S+M180V+E171V | ++++ |
| F88A+L59A+Y89D+N86H+Y85M+K90G+T91M+P83S+V234A+L380A+S417I+A168E+A3 79L+S424A+F301S+G164S+T452S+M180V+F449L | ++++ |
| F88A+L59A+Y89D+N86H+Y85M+K90G+T91 M+P83S+V234A+L380A+S417I+A168E+A3 79L+S424A+F301S+G164S+T452S+M180V+F320M | ++++ |
| F88A+L59A+Y89D+N86H+Y85M+K90G+T91 M+P83S+V234A+L380A+S417I+A168E+A3 79L+S424A+F301S+G164S+T452S+M180V+F320H | ++++ |
| F88A+L59A+Y89D+N86H+Y85M+K90G+T91 M+P83S+V234A+L380A+S417I+A168E+A3 79L+S424A+F301S+G164S+T452S+M180V+Y322N | ++++ |
| F88A+L59A+Y89D+N86H+Y85M+K90G+T91 M+P83S+V234A+L380A+S417I+A168E+A3 79L+S424A+F301S+G164S+T452S+M180V+Y322T | ++++ |
| F88A+L59A+Y89D+N86H+Y85M+K90G+T91M+P83S+V234A+L380A+S417I+A168E+A3 79L+S424A+F301S+G164S+T452S+M180V+F449L+F320H+Y322C | ++++ |
| F88A+L59A+Y89D+N86H+Y85M+K90G+T91 M+P83S+V234A+L380A+S417I+A168E+A3 79L+S424A+F301S+G164S+T452S+M180V+F449L+F320H+Y322T | ++++ |
| F88A+L59A+Y89D+N86H+Y85M+K90G+T91M+P83S+V234A+L380A+S417I+A168E+A3 79L+S424A+F301S+G164S+T452S+M180V+F449L+F320H+Y322S | ++++ |
| F88A+L59A+Y89D+N86H+Y85M+K90G+T91M+P83S+V234A+L380A+S417I+A168E+A3 79L+S424A+F301S+G164S+T452S+M180V+F449L+Y322S | ++++ |
| F88A+L59A+Y89D+N86H+Y85M+K90G+T91M+P83S+V234A+L380A+S417I+A168E+A3 79L+S424A+F301S+G164S+T452S+M180V+F449L+Y322T | ++++ |
| F88A+L59A+Y89D+N86H+Y85M+K90G+T91M+P83S+V234A+L380A+S417I+A168E+A3 79L+S424A+F301S+G164S+T452S+M180V+F449L+F320H+Y322T+M91I | ++++ |
| F88A+L59A+Y89D+N86H+Y85M+K90G+T91M+P83S+V234A+L380A+S417I+A168E+A3 79L+S424A+F301S+G164S+T452S+M180V+F449L+F320H+Y322T+D315V | ++++ |

| | |
|---|---|
| Note: in the above table, + represented the conversion rate was greater than or equal to 0% and less than 20%, ++ represented the conversion rate was greater than or equal to 20% and less than 40%, +++ represented the conversion rate was greater than or equal to 40% and less than 80%, and ++++ represented the conversion rate was greater than or equal to 80%. | |

### Embodiment 10

50 mL of 100 mmol/L phosphate buffer (5 vol) and 20 mL of 5 mol/L isopropylamine hydrochloride solution (2 vol) were added to a 250 mL four-port flask at a room temperature, and the pH was adjusted to 8.5-9.0. 0.1 g of pyridoxal phosphate (1wt%), and 10 g of 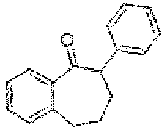 (Substrate 1) were added, and stirred evenly, and then 10 mL of enzyme solution (0.5 wt, 0.5 g/mL) of a CvTA transaminase mutant (F88A+L59A+Y89D+N86H+Y85M+K90G+T91M+P83S+V234A+L380A+S417I) mutated on the basis of SEQ ID NO: 2 was added, and the pH was adjusted to 8.5-9.0. The temperature was raised to 50°C, and it was stirred and reacted overnight.. After the reaction was completed, the system was adjusted with the acid to pH=2-3, denatured protein. After being filtered, a filtrate was extracted with 50 mL methyl tert-butyl ether. A aqueous phase was adjusted to pH=12, and then 50 mL of methyl tert-butyl ether was used for extraction twice. The combined organic phase was dried with anhydrous magnesium sulfate and concentrated to no fraction under the conditions of T<40°C and P≤-0.06 Mpa. A target product 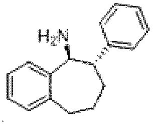 was obtained.

After being detected by high performance liquid chromatography (HPLC), the purity is >98%, the diastereomeric excess (de) value is >99%, and the yield is 84%.

### Embodiment 11

50 mL of 100 mmol/L phosphate buffer (5 vol) and 20 mL of 5 mol/L isopropylamine hydrochloride solution (2 vol) were added to a 250 mL four-port flask at a room temperature, and the pH was adjusted to 8.5-9.0. 0.1 g of pyridoxal phosphate (1wt%), and 10 g of 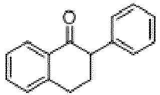 (Substrate 2) were added, and stirred evenly, and then 10 mL of enzyme solution (0.5 wt, 0.5 g/mL) of a CvTA transaminase mutant (F88A+L59A+Y89D+N86H+Y85M+K90G+T91M+P83S+V234A+L380A+S417I) mutated on the basis of SEQ ID NO: 2 was added, and the pH was adjusted to 8.5-9.0. The temperature was raised to 50 °C, and it was stirred and reacted overnight. After the reaction was completed, the system was adjusted with acid that pH=2-3, and a protein was denatured. After being filtered, a filtrate was extracted with 50 mL of methyl tert-butyl ether. An aqueous phase was adjusted to pH=12, and then 50 mL of methyl tert-butyl ether was used for extraction twice. The combined organic phase was dried with anhydrous magnesium sulfate and concentrated to no fraction under the conditions of T<40 °C and P≤-0.06 Mpa. A target product 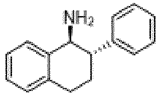 was obtained.

After being detected by HPLC, the purity was >98%, the de value was >99%, and the yield was 82%.

### Embodiment 12

50 mL of 100 mmol/L phosphate buffer (5 vol) and 20 mL of 5 mol/L isopropylamine hydrochloride solution (2 vol) were added to a 250 mL four-port flask at a room temperature, and the pH was adjusted to 8.5-9.0. 0.1 g of pyridoxal phosphate (1wt%), and 10 g of 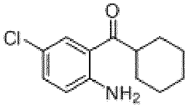 (Substrate 3) were added, and stirred evenly, and then 10 mL of enzyme solution (0.5 wt, 0.5 g/mL) of a CvTA transaminase mutant (F88A+L59A+Y89D+N86H+Y85M+K90G+T91M+P83S+V234A+L380A+S417I) mutated on the basis of SEQ ID NO: 2 was added, and the pH was adjusted to 8.5-9.0. The temperature was raised to 50°C, and it was stirred and reacted overnight. After the reaction was completed, the system was adjusted with acid that pH=2-3, and a protein was denatured. After being filtered, a filtrate was extracted with 50 mL of methyl tert-butyl ether. An aqueous phase was adjusted to pH=12, and then 50 mL of methyl tert-butyl ether was used for extraction twice. The combined organic phase was dried with anhydrous magnesium sulfate and concentrated to no fraction under the conditions of T<40 °C and P≤-0.06 Mpa. A target product 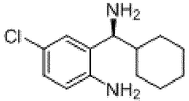 was obtained.

After being detected by HPLC, the purity was >98%, the de value was >99%, and the yield was 80%.

### Embodiment 13

50 mL of 100 mmol/L phosphate buffer (5 vol) and 20 mL of 5 mol/L isopropylamine hydrochloride solution (2 vol) were added to a 250 mL four-port flask at a room temperature, and the pH was adjusted to 8.5-9.0. 0.1 g of pyridoxal phosphate (1wt%), and 10 g of 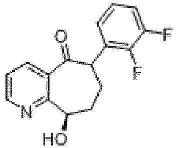 (Substrate 4) were added, and stirred evenly, and then 10 mL of enzyme solution (0.5 wt, 0.5 g/mL) of a CvTA transaminase mutant (F88A+L59A+Y89D+N86H+Y85M+K90G+T91M+P83S+V234A+L380A+S417I) mutated on the basis of SEQ ID NO: 2 was added, and the pH was adjusted to 8.5-9.0. The temperature was raised to 50 °C, and it was stirred and reacted overnight. After the reaction was completed, the system was adjusted with acid that pH=2-3, and a protein was denatured. After being filtered, a filtrate was extracted with 50 mL of methyl tert-butyl ether. An aqueous phase was adjusted to pH=12, and then 50 mL of methyl tert-butyl ether was used for extraction twice. The combined organic phase was dried with anhydrous magnesium sulfate and concentrated to no fraction under the conditions of T<40 °C and P≤-0.06 Mpa. A target product 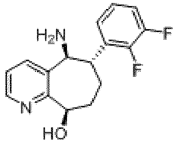 was obtained.

After being detected by HPLC, the purity was >98%, the de value was >99%, and the yield was 89%.

### Embodiment 14

50 mL of 100 mmol/L phosphate buffer (5 vol) and 20 mL of 5 mol/L isopropylamine hydrochloride solution (2 vol) were added to a 250 mL four-port flask at a room temperature, and the pH was adjusted to 8.5-9.0. 0.1 g of pyridoxal phosphate (1wt%), and 10 g of 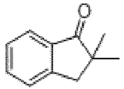 (Substrate 13) were added, and stirred evenly, and then 10 mL of enzyme solution (0.5 wt, 0.5 g/mL) of a CvTA transaminase mutant (F88A+L59A+Y89D+N86H+Y85M+K90G+T91M+P83S+V234A+L380A+S417I) mutated on the basis of SEQ ID NO: 2 was added, and the pH was adjusted to 8.5-9.0. The temperature was raised to 50 °C, and it was stirred and reacted overnight. After the reaction was completed, the system was adjusted with acid that pH=2-3, and a protein was denatured. After being filtered, a filtrate was extracted with 50 mL of methyl tert-butyl ether. An aqueous phase was adjusted to pH=12, and then 50 mL of methyl tert-butyl ether was used for extraction twice. The combined organic phase was dried with anhydrous magnesium sulfate and concentrated to no fraction under the conditions of T<40 °C and P≤-0.06 Mpa. A target product 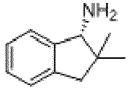 was obtained.

After being detected by HPLC, the purity was >98%, the ee value was >98%, and the yield was 81%.

### Embodiment 15

Enzyme solution of a CvTA transaminase mutant (F88A+L59A+Y89D+N86H+Y85M+K90G+T91M+P83S+V234A+L380A+S417I+A168E+A379L+S4 24A+F301S+G164S+T452S+M180V+F449L+F320H+Y322T+M911) mutated on the basis of SEQ ID NO: 2 was used, the catalytic synthesis steps of Embodiments 10 to 14 were referenced, a catalytic reaction was performed on the corresponding substrates, and results were respectively as follows.
(1) The purity of the target product synthesized by catalyzing Substrate 1 was >99%, the de value was >99%, and the yield was 87%;
(2) the purity of the target product synthesized by catalyzing Substrate 2 was >98%, the de value was >99%, and the yield was 84%;
(3) the purity of the target product synthesized by catalyzing Substrate 3 was >98%, the ee value was >99%, and the yield was 82%;
(4) the purity of the target product synthesized by catalyzing Substrate 4 was >99%, the de value was >99%, and the yield was 91%; and
(5) the purity of the target product synthesized by catalyzing Substrate 13 was >98%, the ee value was >99%, and the yield was 85%.

From the description of the above embodiments, it may be seen that the above embodiments achieve the following technical effects.
1) A series of chiral amine compounds with high activity and high selectivity may be obtained by using the transaminase with improved activity to transaminate.
2) The use of the transaminase mutant for biocatalysis does not require the use of heavy metal catalysts, as to achieve the green chemistry.
3) The transaminase mutant has the high catalytic efficiency, small reaction volume and high product yield, the three wastes are greatly reduced, and the production cost is saved.

(II) Part two: catalytic activities of transaminase mutants from different species sources on such large sterically hindered substrates are further screened and verified.

### Embodiment 16: Catalytic activity test of wild-type transaminase from other sources

In this embodiment, the wild-type transaminases (specifically shown in Table 10), which belong to the same genus but are different species from the wild-type transaminase (namely SEQ ID NO: 1) derived from *Chromobaterium violaceum,* and have more than 82% identity with the amino acid sequence of SEQ ID NO: 1, was selected, and its catalytic activity on Substrate 4 was tested. Results were similar to that of the CvTA wild-type transaminase (less than 1%), and the specific results were shown in Table 11.

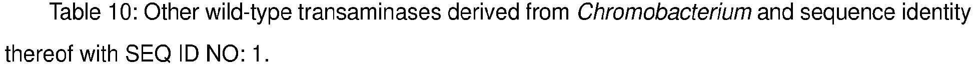

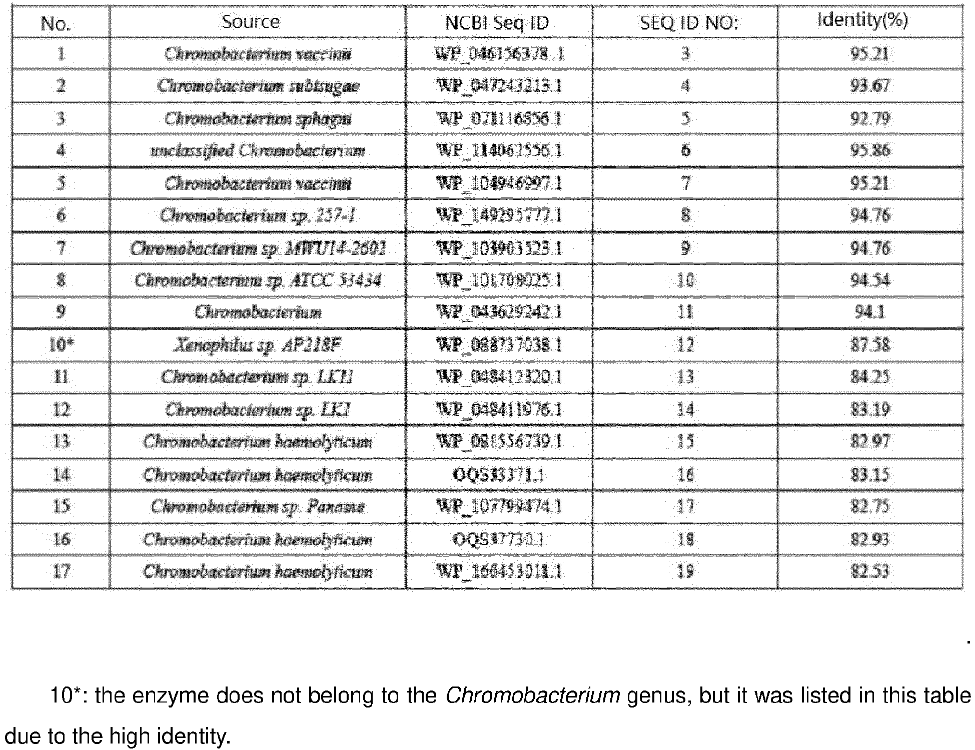

### Embodiment 17: Screening of target large sterically hindered substrate catalytic activity mutants

In this embodiment, a L59A mutation was performed on the wild-type transaminase in the 59-th site corresponding to SEQ ID NO: 1 in Table 10 above, and at the same time, any one or more mutations of Y85M, N86H, F88A and F89D were performed, which improved the catalytic activity of the target large sterically hindered substrate (Substrate 4) in different degrees (shown in Table 11). This result showed that the transaminase, which has 82% to 95% of the identity with SEQ ID NO: 1, improved the catalytic activity on the large sterically hindered substrate after the same amino acid mutation occurred at the conserved amino acid sites.

**Table 11:**

| Enzyme number | Mutant activity (conversion rate of Substrate 4) | | | |
|---|---|---|---|---|
| | F59A+F88A | F59A+F88A+ F89D | F59A+F88A+ F89D+N86H | F59A+F88A+F89D +N86H+Y85M |
| 1 | + | + | + | ++ |
| 2 | + | + | + | ++ |
| 3 | + | + | + | + |
| 4 | + | + | + | ++ |
| 5 | + | + | + | ++ |
| 6 | + | + | + | + |
| 7 | + | + | + | ++ |
| 8 | + | + | + | + |
| 9 | + | + | + | + |
| 10 | + | + | + | ++ |
| 11 | + | + | + | + |
| 12 | + | + | + | ++ |
| 13 | + | + | + | + |
| 14 | + | + | + | ++ |
| 15 | + | + | + | + |
| 16 | + | + | + | + |
| 17 | + | + | + | + |

In the above table, + represented the conversion rate was between 1-20%, and ++ represented the conversion rate was greater than 20%.

### Embodiment 18

In addition to the transaminase derived from *Chromobacterium,* the present disclosure also tested the transaminases from other sources having 69% to 87% of the identity with *Chromobaterium violaceum.* Similarly, the catalytic activity for Substrate 4 was less than 1%. However, the activities of these transaminases were improved to varying degrees after the same amino acid mutation occurred in the conserved amino acid sequence region as shown in Table 11. Specific information was listed in Tables 12 and 13.

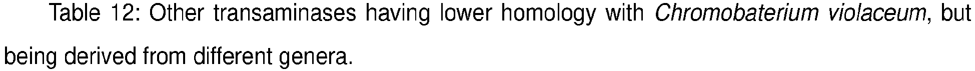

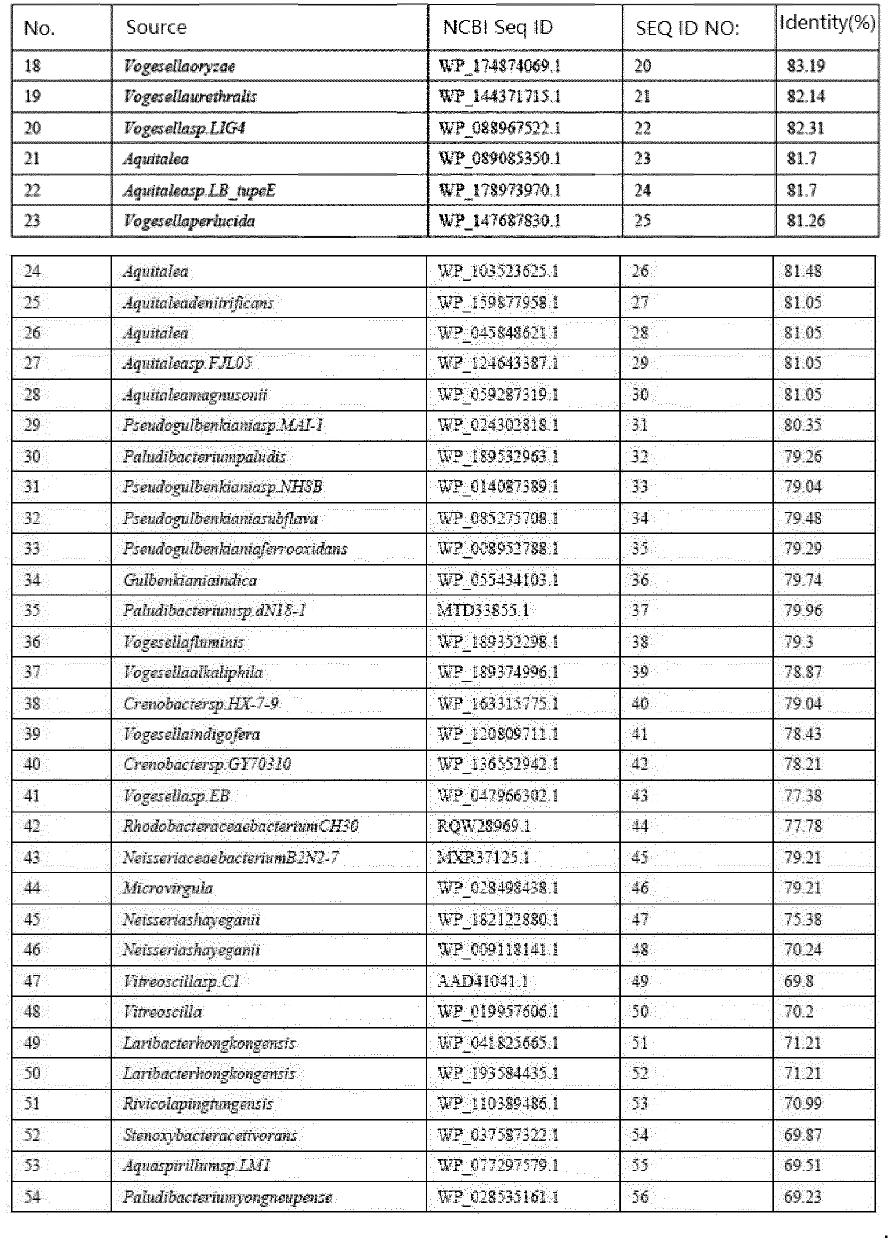

Table 13:

| Enzyme number | Mutant activity (conversion rate of Substrate 4) | | | |
|---|---|---|---|---|
| | F59A+F88A | F59A+F88A+ F89D | F59A+F88A+ F89D+N86H | F59A+F88A+F89D +N86H+Y85M |
| 18 | + | + | + | + |
| 19 | + | + | + | + |
| 20 | + | + | + | + |
| 21 | + | + | + | + |
| 22 | + | + | + | + |
| 23 | + | + | + | + |
| 24 | + | + | + | + |
| 25 | + | + | + | + |
| 26 | + | + | + | + |
| 27 | + | + | + | + |
| 28 | + | + | + | + |
| 29 | + | + | + | + |
| 30 | + | + | + | + |
| 31 | + | + | + | + |
| 32 | + | + | + | + |
| 33 | + | + | + | + |
| 34 | + | + | + | + |
| 35 | + | + | + | + |
| 36 | + | + | + | + |
| 37 | + | + | + | + |
| 38 | + | + | + | + |
| 39 | + | + | + | + |
| 40 | + | + | + | + |
| 41 | + | + | + | + |
| 42 | + | + | + | + |
| 43 | + | + | + | + |
| 44 | + | + | + | + |
| 45 | + | + | + | + |
| 46 | + | + | + | + |
| 47 | + | + | + | + |
| 48 | + | + | + | + |
| 49 | + | + | + | + |
| 50 | + | + | + | + |
| 5i | + | + | + | + |
| 52 | + | + | + | + |
| 53 | + | + | + | + |
| 54 | + | + | + | + |

In the above table, + represented the conversion rate was between 1-20%, and ++ represented the conversion rate was greater than 20%.

### Embodiment 19

50 mL of 100 mmol/L phosphate buffer (5 vol) and 24 mL of 5 mol/L isopropylamine hydrochloride solution (2 vol) were added to a 250 mL four-port flask at a room temperature, and the pH was adjusted to 7.5-8.0. 0.1 g of pyridoxal phosphate (1wt%), and 1 g of 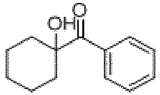 (Substrate 5) were added, and stirred evenly, and then 10 mL of enzyme solution (5 wt, 0.5 g/mL) of a CvTA transaminase mutant (L59A+Y85M+N86H+F88A+F89D) mutated on the basis of SEQ ID NO: 1 was added, and the pH was adjusted to 7.5-8.0. The temperature was raised to 30 °C, and it was stirred and reacted overnight. After the reaction was completed, the system was adjusted with acid to pH=1, and a protein was denatured. After being filtered, a filtrate was extracted with 50 mL of dichloromethane. An aqueous phase was adjusted to pH=12, and then it was extracted twice with 50 mL of the dichloromethane. After a combined organic phase was dried with anhydrous magnesium sulfate, the organic phase was concentrated until there was no fraction under the conditions of T<35°C and P≤-0.06 Mpa. A target product 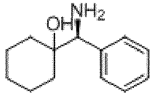 was obtained.

After being detected by HPLC, the purity was >98%, the ee value was >99%, and the yield was 84%.

### Embodiment 20

60 mL of 100 mmol/L phosphate buffer (6 vol) and 18 mL of 5 mol/L isopropylamine hydrochloride solution (2 vol) were added to a 250 mL four-port flask at a room temperature, and the pH was adjusted to 8.5-9.08.0-8.5. 0.1 g of pyridoxal phosphate (1wt%), and 1 g of 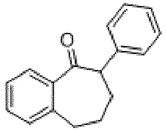 (Substrate 1) were added, and stirred evenly, and then 10 mL of enzyme solution (5 wt, 0.5 g/mL) of a CvTA transaminase mutant (L59A+Y85M+N86H+F88A+F89D) mutated on the basis of SEQ ID NO: 1 was added, and it was adjusted to pH=8.0-8.5. The temperature was raised to 20 °C, and it was stirred and reacted overnight. After the reaction was completed, the system was adjusted with acid to pH=2, and a protein was denatured. After being filtered, a filtrate was extracted with 50 mL of dichloromethane. An aqueous phase was adjusted to pH=13, and then it was extracted for three times with 50 mL of ethyl acetate. After a combined organic phase was dried with anhydrous magnesium sulfate, the organic phase was concentrated until there was no fraction under the conditions of T<45°C and P≤-0.08 Mpa. A target product 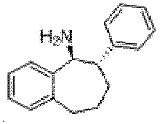 was obtained.

After being detected by HPLC, the purity was >98%, the de value was >99%, and the yield was 89%.

### Embodiment 21

70 mL of 100 mmol/L phosphate buffer (7 vol) and 13 mL of 5 mol/L isopropylamine hydrochloride solution (2 vol) were added to a 250 mL four-port flask at a room temperature, and the pH was adjusted to 7.0-7.5. 0.1 g of pyridoxal phosphate (1wt%), and 1 g of 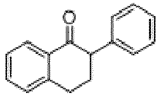 (Substrate 2) were added, and stirred evenly, and then 10 mL of enzyme solution (5 wt, 0.5 g/mL) of a CvTA transaminase mutant (L59A+Y85M+N86H+F88A+F89D) mutated on the basis of SEQ ID NO: 1 was added. The temperature was raised to 40°C, and it was stirred and reacted overnight. After the reaction was completed, the system was adjusted with acid to pH=1, and a protein was denatured. After being filtered, a filtrate was extracted with 50 mL of dichloromethane. An aqueous phase was adjusted to pH=13, and then it was extracted for three times with 50 mL of the dichloromethane. After a combined organic phase was dried with anhydrous magnesium sulfate, the organic phase was concentrated until there was no fraction under the conditions of T<35°C and P≤-0.06 Mpa. A target product 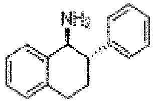 was obtained.

After being detected by HPLC, the purity was greater than 97%, the de value was >99%, and the yield was 86%.

### Embodiment 22

50 mL of 100 mmol/L phosphate buffer (5 vol) and 38 mL of 5 mol/L isopropylamine hydrochloride solution (2 vol) were added to a 250 mL four-port flask at a room temperature, and the pH was adjusted to 8.5-9.0. 0.1 g of pyridoxal phosphate (1wt%), and 1 g of 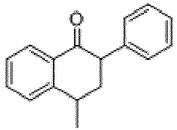 (Substrate 6) were added, and stirred evenly, and then 10 mL of enzyme solution (5 wt, 0.5 g/mL) of a CvTA transaminase mutant (L59A+Y85M+N86H+F88A+F89D) mutated on the basis of SEQ ID NO: 1 was added, and the pH was adjusted to 8.5-9.0. The temperature was raised to 10 °C, and it was stirred and reacted overnight. After the reaction was completed, the system was adjusted with acid that pH=2, and a protein was denatured. After being filtered, a filtrate was extracted with 50 mL of dichloromethane. An aqueous phase was adjusted to pH=13, and then it was extracted for three times with 50 mL of the dichloromethane. After a combined organic phase was dried with anhydrous magnesium sulfate, the organic phase was concentrated until there was no fraction under the conditions of T<35°C and P≤-0.06 Mpa. A target product 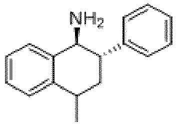 was obtained.

After being detected by HPLC, the purity was >95%, the de value was >99%, and the yield was 85%.

### Embodiment 23

70 mL of 100 mmol/L phosphate buffer (7 vol) and 17 mL of 5 mol/L isopropylamine hydrochloride solution (2 vol) were added to a 250 mL four-port flask at a room temperature, and the pH was adjusted to 7.5-8.0. 0.1 g of pyridoxal phosphate (1wt%), and 1 g of 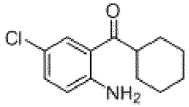 (Substrate 3) were added, and stirred evenly, and then 10 mL of enzyme solution (5 wt, 0.5 g/mL) of a CvTA transaminase mutant (L59A+Y85M+N86H+F88A+F89D) mutated on the basis of SEQ ID NO: 1 was added, and the pH was adjusted to 7.5-8.0. The temperature was raised to 29 °C, and it was stirred and reacted overnight. After the reaction was completed, the system was adjusted with acid to pH=1, and a protein was denatured. After being filtered, a filtrate was extracted with 50 mL of dichloromethane. An aqueous phase was adjusted to pH=12, and then it was extracted for three times with 50 mL of ethyl acetate. After a combined organic phase was dried with anhydrous magnesium sulfate, the organic phase was concentrated until there was no fraction under the conditions of T<45°C and P≤-0.08 Mpa. A target product 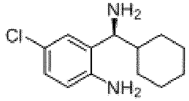 was obtained.

After being detected by HPLC, the purity was >96%, the ee value was >99%, and the yield was 77%.

### Embodiment 24

140 mL of 100 mmol/L phosphate buffer (7 vol) and 50 mL of 5 mol/L isopropylamine hydrochloride solution (2 vol) were added to a 500 mL four-port flask at a room temperature, and the pH was adjusted to 7.5-8.0. 0.2 g of pyridoxal phosphate (1wt%), and 2 g of 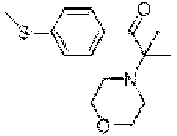 (Substrate 7) were added, and stirred evenly, and then 20 mL of enzyme solution (5 wt, 0.5 g/mL) of a CvTA transaminase mutant (L59A+Y85M+N86H+F88A+F89D) mutated on the basis of SEQ ID NO: 1 was added, and it was adjusted to pH=7.5-8.0. The temperature was raised to 32°C, and it was stirred and reacted overnight. After the reaction was completed, the system was adjusted with acid that pH=1, and a protein was denatured. After being filtered, a filtrate was extracted with 100 mL of dichloromethane. An aqueous phase was adjusted to pH=12, and then it was extracted for three times with 100 mL of ethyl acetate. After a combined organic phase was dried with anhydrous magnesium sulfate, the organic phase was concentrated until there was no fraction under the conditions of T<45°C and P≤-0.08 Mpa. A target product 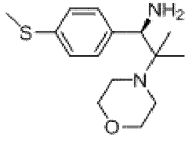 was obtained.

After being detected by HPLC, the purity was >95%, the ee value was >99%, and the yield was 80%.

### Embodiment 25

220 mL of 100 mmol/L phosphate buffer (11 vol) and 50 mL of 5 mol/L isopropylamine hydrochloride solution (2 vol) were added to a 500 mL four-port flask at a room temperature, and it was adjusted to pH=7.5-8.0. 0.2 g of pyridoxal phosphate (1wt%), and 2 g of 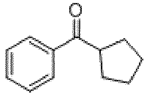 (Substrate 8) were added, and stirred evenly, and then 40 mL of enzyme solution (10 wt, 0.5 g/mL) of a CvTA transaminase mutant (L59A+Y85M+N86H+F88A+F89D) mutated on the basis of SEQ ID NO: 1 was added, and it was adjusted to pH=7.5-8.0. The temperature was raised to 32°C, and it was stirred and reacted overnight. After the reaction was completed, the system was adjusted with acid that pH=1, and a protein was denatured. After being filtered, a filtrate was extracted with 100 mL of dichloromethane. An aqueous phase was adjusted to pH=12, and then it was extracted for three times with 100 mL of ethyl acetate. After a combined organic phase was dried with anhydrous magnesium sulfate, the organic phase was concentrated until there was no fraction under the conditions of T<45°C and P≤-0.08 Mpa. A target product 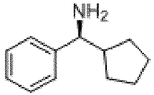 was obtained.

After being detected by HPLC, the purity was >95%, the ee value was >99%, and the yield was 86%.

### Embodiment 26

180 mL of 100 mmol/L phosphate buffer (12 vol) and 36 mL of 5 mol/L isopropylamine hydrochloride solution (2 vol) were added to a 500 mL four-port flask at a room temperature, and it was adjusted to pH=7.5-8.0. 0.15 g of pyridoxal phosphate (1wt%), and 1.5 g of 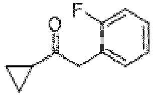 (Substrate 9) were added, and stirred evenly, and then 30 mL of enzyme solution (1 wt, 0.5 g/mL) of a CvTA transaminase mutant (L59A+Y85M+N86H+F88A+F89D) mutated on the basis of SEQ ID NO: 1 was added, and it was adjusted to pH=7.5-8.0. The temperature was raised to 35°C, and it was stirred and reacted overnight. After the reaction was completed, the system was adjusted with acid that pH=1, and a protein was denatured. After being filtered, a filtrate was extracted with 100 mL of dichloromethane. An aqueous phase was adjusted to pH=12, and then it was extracted for three times with 100 mL of ethyl acetate. After a combined organic phase was dried with anhydrous magnesium sulfate, the organic phase was concentrated until there was no fraction under the conditions of T<45°C and P≤-0.08 Mpa. A target product 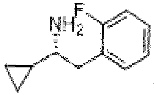 was obtained.

After being detected by HPLC, the purity was >96%, the ee value was >99%, and the yield was 85%.

### Embodiment 27

180 mL of 100 mmol/L phosphate buffer (12 vol) and 26 mL of 5 mol/L isopropylamine hydrochloride solution (2 vol) were added to a 500 mL four-port flask at a room temperature, and the pH was adjusted to 7.5-8.0. 0.15 g of pyridoxal phosphate (1wt%), and 1.5 g of 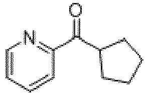 (Substrate 10) were added, and stirred evenly, and then 30 mL of enzyme solution (10 wt, 0.5 g/mL) of a CvTA transaminase mutant (L59A+Y85M+N86H+F88A+F89D) mutated on the basis of SEQ ID NO: 1 was added, and the pH was adjusted to7.5-8.0. The temperature was raised to 40°C, and it was stirred and reacted overnight. After the reaction was completed, the system was adjusted with acid that pH=1, and a protein was denatured. After being filtered, a filtrate was extracted with 100 mL of dichloromethane. An aqueous phase was adjusted to pH=12, and then it was extracted for three times with 100 mL of ethyl acetate. After a combined organic phase was dried with anhydrous magnesium sulfate, the organic phase was concentrated until there was no fraction under the conditions of T<45°C and P≤-0.08 Mpa. A target product 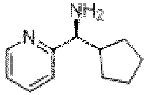 was obtained.

After being detected by HPLC, the purity was >97%, the ee value was >99%, and the yield was 79%.

### Embodiment 28

110 mL of 100 mmol/L phosphate buffer (11 vol) and 23 mL of 5 mol/L isopropylamine hydrochloride solution (2 vol) were added to a 500 mL four-port flask at a room temperature, and the pH was adjusted to7.5-8.0. 0.1 g of pyridoxal phosphate (1wt%), and 1 g of 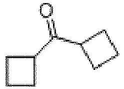 (Substrate 11) were added, and stirred evenly, and then 10 mL of enzyme solution (5 wt, 0.5 g/mL) of a CvTA transaminase mutant (L59A+Y85M+N86H+F88A+F89D) mutated on the basis of SEQ ID NO: 1 was added, and the pH was adjusted to7.5-8.0. The temperature was raised to 40°C, and it was stirred and reacted overnight. After the reaction was completed, the system was adjusted with acid that pH=2, and a protein was denatured. After being filtered, a filtrate was extracted with 50 mL of dichloromethane. An aqueous phase was adjusted to pH=12, and then it was extracted for three times with 50 mL of ethyl acetate. After a combined organic phase was dried with anhydrous magnesium sulfate, the organic phase was concentrated until there was no fraction under the conditions of T<45°C and P≤-0.08 Mpa. A target product 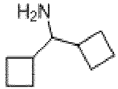 was obtained.

After being detected by HPLC, the purity was >95%, and the yield was 73%.

### Embodiment 29

110 mL of 100 mmol/L phosphate buffer (11 vol) and 23 mL of 5 mol/L isopropylamine hydrochloride solution (2 vol) were added to a 500 mL four-port flask at a room temperature, and the pH was adjusted to7.5-8.0. 0.1 g of pyridoxal phosphate (1wt%), and 1 g of 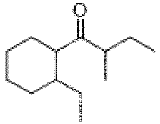 (Substrate 12) were added, and stirred evenly, and then 10 mL of enzyme solution (5 wt, 0.5 g/mL) of a CvTA transaminase mutant (L59A+Y85M+N86H+F88A+F89D) mutated on the basis of SEQ ID NO: 1 was added, and the pH was adjusted to7.5-8.0. The temperature was raised to 40°C, and it was stirred and reacted overnight. After the reaction was completed, the system was adjusted with acid that pH=2, and a protein was denatured. After being filtered, a filtrate was extracted with 50 mL of dichloromethane. An aqueous phase was adjusted to pH=12, and then it was extracted for three times with 50 mL of ethyl acetate. After a combined organic phase was dried with anhydrous magnesium sulfate, the organic phase was concentrated until there was no fraction under the conditions of T<45°C and P≤-0.08 Mpa. A target product 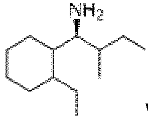 was obtained.

After being detected by HPLC, the purity was >95%, the ee value was >99%, and the yield was 67%.

### Embodiment 30

110 mL of 100 mmol/L phosphate buffer (11 vol) and 23 mL of 5 mol/L isopropylamine hydrochloride solution (2 vol) were added to a 500 mL four-port flask at a room temperature, and the pH was adjusted to7.5-8.0. 0.1 g of pyridoxal phosphate (1wt%), and 1 g of 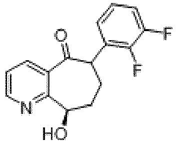 (Substrate 4) were added, and stirred evenly, and then 10 mL of enzyme solution (5 wt, 0.5 g/mL) of a CvTA transaminase mutant (L59A+Y85M+N86H+F88A+F89D) mutated on the basis of SEQ ID NO: 1 was added, and the pH was adjusted to7.5-8.0. The temperature was raised to 40°C, and it was stirred and reacted overnight. After the reaction was completed, the system was adjusted with acid that pH=2, and a protein was denatured. After being filtered, a filtrate was extracted with 50 mL of dichloromethane. An aqueous phase was adjusted to pH=12, and then it was extracted for three times with 50 mL of ethyl acetate. After a combined organic phase was dried with anhydrous magnesium sulfate, the organic phase was concentrated until there was no fraction under the conditions of T<45°C and P≤-0.08 Mpa. A target product 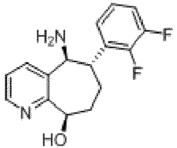 was obtained.

After being detected by HPLC, the purity was >95%, the de value was >99%, and the yield was 80%.

### Embodiment 31

50 mL of 100 mmol/L phosphate buffer (5 vol) and 20 mL of 5 mol/L isopropylamine hydrochloride solution (2 vol) were added to a 250 mL four-port flask at a room temperature, and the pH was adjusted to 8.5-9.0. 0.1 g of pyridoxal phosphate (1wt%), and 1 g of 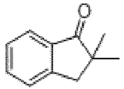 (Substrate 13) were added, and stirred evenly, and then 10 mL of enzyme solution (0.5 wt, 0.5 g/mL) of a CvTA transaminase mutant (L59A+Y85M+N86H+F88A+F89D) mutated on the basis of SEQ ID NO: 1 was added, and the pH was adjusted to 8.5-9.0. The temperature was raised to 40 °C, and it was stirred and reacted overnight. After the reaction was completed, the system was adjusted with acid that pH=2-3, and a protein was denatured. After being filtered, a filtrate was extracted with 50 mL of methyl tert-butyl ether. An aqueous phase was adjusted to pH=12, and then 50 mL of methyl tert-butyl ether was used for extraction twice. After a combined organic phase was dried with anhydrous magnesium sulfate, the organic phase was concentrated until there was no fraction under the conditions of T<40°C and P≤-0.06 Mpa. A target product 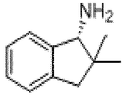 was obtained.

After being detected by HPLC, the purity was 93%, the ee value was 98%, and the yield was 73%.

### Embodiment 32

Substrate 1 was taken as an example, the enzyme amount of the reaction was optimized, and specific operations were as follows.

1.2 mL of 100 mmol/L phosphate buffer (6 vol) and 0.4 mL of 5 mol/L isopropylamine hydrochloride solution (2 vol) were added to a 10 mL vial at a room temperature, and the pH was adjusted to8.0-8.5. 0.002 g of pyridoxal phosphate (1wt%), and 0.02 g of 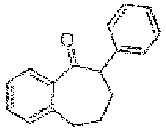 (Substrate 1) were added, and mixed evenly, and 0.04 mL, 0.08 mL, 0.12 mL, 0.16 mL, 0.2 mL, 0.24 mL, 0.28 mL, 0.32 mL, 0.36 mL, and 0.4 mL of enzyme solution (the concentration of the enzyme solution was0.5 g/mL, and the corresponding mass ratios were 1 wt, 2 wt, 3 wt, 4 wt, 5 wt, 6 wt, 7 wt, 8 wt, 9 wt, and 10 wt, respectively.) of a CvTA mutant (L59A+Y85M+N86H+F88A+F89D) were added, and the pH was adjusted to8.0-8.5. The rotation speed of a shaker was 170 rpm, and it was reacted overnight under the condition of raising the temperature to 30°C. Conversion rate results were shown in the table below, and it was proved that the conversion rate of 5 wt of the substrate might be greater than 99%, and there was no significant effect if the enzyme amount continued to be increased.

**Table 14: Relationship between enzyme amount and conversion rate**

| Enzyme amount (wt) | Conversion rate (%) |
|---|---|
| 1 | 22% |
| 2 | 40% |
| 3 | 70% |
| 4 | 89% |
| 5 | 99% |
| 6 | 99% |
| 7 | 99% |
| 8 | 99% |
| 9 | 99% |
| 10 | 99% |

### Embodiment 33

Substrate 1 was taken as an example, and the substrate concentration of the reaction was optimized. Specific operations were as follows.

0.5 mL of 100 mmol/L phosphate buffer and 0.4 mL of 5 mol/L isopropylamine hydrochloride solution (2 vol) were added to a 10-50 mL vial (an appropriate shaking flask was selected according to the reaction volume) at a room temperature, and the pH was adjusted to8.0-8.5. 0.002 g of pyridoxal phosphate (1wt%), and 0.02 g of 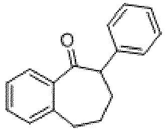 (1 vol, 0.02 ml) were added, and mixed evenly, and 0.2 mL of enzyme solution (1 vol, 5 wt, and the concentration of the enzyme solution was 0.5 g/ml) of a CvTA mutant (L59A+Y85M+N86H+F88A+F89D) was added, 100 mmol/L phosphate buffer solution was supplemented until the reaction volumes were 2 mL, 2.2 mL, 2.4 mL, 3 mL, 3.4 mL, and 4 mL respectively, which were 100 vol, 110 vol, 120 vol, 150 vol, 170 vol, and 200 vol respectively corresponding to the reaction volumes. The pH was adjusted to8.0-8.5. The rotation speed of a shaker was 170 rpm, and it was reacted overnight under the condition of raising the temperature to 30 °C. As shown in the table below, it was proved that under the concentration of 5-10 g/L, the substrate conversion rate might be greater than 99%. The continuous reduction of the substrate concentration might lead to an increase in the reaction volume and an increase in the amount of three wastes.

**Table 15: Relationship between reaction volume and conversion rate**

| Reaction volume (vol) | Conversion rate (%) |
|---|---|
| 100 | 99 |
| 110 | 99 |
| 120 | 99 |
| 150 | 99 |
| 170 | 99 |
| 200 | 99 |

### Embodiment 34

Substrate 1 was taken as an example, the amino donor of the reaction was optimized, and specific operations were as follows.

1.2 mL of 100mmol/L phosphate buffer solution (6 vol) was added to a 10 mL vial at a room temperature, 3 vol of an amino donor system (the amino donors were isopropylamine, isopropylamine hydrochloride, alanine, aniline, and n-butylamine respectively) was used respectively, and the pH was adjusted to 8.0-8.5. 0.002 g of pyridoxal phosphate (1wt%), and 0.02 g of 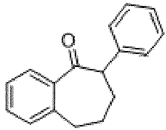 were added, and mixed evenly. 0.2 ml of enzyme solution (5 wt, the concentration of the enzyme solution was 0.5 g/ml) of a CvTA mutant (L59A+Y85M+N86H+F88A+F89D) was added, and the pH was adjusted to 8.0-8.5. The rotation speed of a shaker was 170 rpm, and it was reacted overnight under the condition of raising the temperature to 29 °C. Conversion rate results were shown in the table below, and it was proved that the isopropylamine, isopropylamine hydrochloride, alanine, and n-butylamine were used as the amino donors, and the substrate conversion rate might be greater than 99%. However, the aniline was used as the amino donor, and under this reaction condition, the conversion rate was only 75%.

**Table 16: Effects of amino donors on reaction**

| Amino donor | Conversion rate (%) |
|---|---|
| Isopropylamine | 99% |
| Isopropylamine hydrochloride | 99% |
| Alanine | 99% |
| N-butylamine | 99% |
| Aniline | 75% |

From the above description, it may be seen that the above embodiments of the present disclosure achieve the following technical effects.
1) The transaminases from the different sources are used, to obtain the transaminase mutants after the conserved site mutation, and a biotransformation reaction of a variety of the ketone substrates is catalyzed, to obtain the required target compounds by a one-step reaction, and the substrate types are widely used.
2) The biotransformation reaction using the transaminase has the high selectivity, and the ee or de value of the product is greater than 99%.
3) The above mutated transaminase mutants from the different sources are used for the catalytic reaction, the substrate concentration may reach 100 g/L, and the production efficiency is greatly improved.
4) The transaminase is used for biocatalysis, and the heavy metal catalyst does not need to be used, thus the green chemistry is achieved.
5) The transaminase is high in catalytic efficiency, small in reaction volume, short in synthesis route and high in product yield, the three wastes are greatly reduced, and the production cost is saved.

The above are only preferred embodiment of the present disclosure, and are not intended to limit the present disclosure. For those skilled in the art, the present disclosure may have various variations and changes. Any modifications, equivalent replacements, improvements and the like made within the spirit and principles of the present disclosure shall be included within the scope of protection of the present disclosure.

## Claims

1. A transaminase mutant, wherein the transaminase mutant is selected from:
(1) a mutant obtained from a mutation of amino acids of SEQ ID NO: 2, the mutation of amino acids is selected from any one of the group consisting of:
| | | |
|---|---|---|
| F22A | F88A+L59H | F88A+L59A+Y89C |
| L59A | F88A+L59R | F88A+L59A+Y89T |
| F88A | F88A+L59F | F88A+L59A+Y89K |
| Y89A | F88A+L59Y | F88A+L59A+Y89D+T87G |
| K90A | F88A+L59S | F88A+L59A+Y89D+T87L |
| T107A | F88A+L59C | F88A+L59A+Y89D+T87S |
| N151A | F88A+L59Q | F88A+L59A+Y89D+T87V |
| Y153A | F88A+L59M | F88A+L59A+Y89D+T87P |
| F166A | F88A+L59L | F88A+L59A+Y89D+T87A |
| E171A | F88A+L59V | F88A+L59A+Y89D+T87W |
| K193A | F88A+L59G | F88A+L59A+Y89D+T87M |
| V234A | F88A+L59A | F88A+L59A+Y89D+T87I |
| I262A | F88A+L59D | F88A+L59A+Y89D+T87D |
| I297A | F88A+L59N | F88A+L59A+Y89D+T87R |
| K304A | F88A+L59R | F88A+L59A+Y89D+T87F |
| F320A | F88A+L59W | F88A+L59A+Y89D+T87H |
| T321A | F88A+L59P | F88A+L59A+Y89D+T87Q |
| E368A | F88A+L59A+Y89G | F88A+L59A+Y89D+T87N |
| L380A | F88A+L59A+Y89L | F88A+L59A+Y89D+T87E |
| F397A | F88A+L59A+Y89S | F88A+L59A+Y89D+T87C |
| R405A | F88A+L59A+Y89V | F88A+L59A+Y89D+T87K |
| F409A | F88A+L59A+Y89P | F88A+L59A+Y89D+N86G |
| D416A | F88A+L59A+Y89A | F88A+L59A+Y89D+N86C |
| S417A | F88A+L59A+Y89W | F88A+L59A+Y89D+N86S |
| C418A | F88A+L59A+Y89M | F88A+L59A+Y89D+N86R |
| S424A | F88A+L59A+Y89I | F88A+L59A+Y89D+N86V |
| / | F88A+L59A+Y89D | F88A+L59A+Y89D+N86F |
| / | F88A+L59A+Y89R | F88A+L59A+Y89D+N86W |
| / | F88A+L59A+Y89F | F88A+L59A+Y89D+N86T |
| / | F88A+L59A+Y89H | F88A+L59A+Y89D+N86I |
| / | F88A+L59A+Y89Q | F88A+L59A+Y89D+N86M |
| / | F88A+L59A+Y89N | F88A+L59A+Y89D+N86L |
| / | F88A+L59A+Y89E | F88A+L59A+Y89D+N86A |
| F88A+L59A+Y89D+N86E | F88A+L59A+Y89D+N86Q | F88A+L59A+Y89D+N86Y |
| | F88A+L59A+Y89D+N86P | F88A+L59A+Y89D+N86K |
| | F88A+L59A+Y89D+N86H | F88A+L59A+Y89D+N86D |
| | |
|---|---|
| F88A+L59A+Y89D+N86H+F84S | F88A+L59A+Y89D+N86H+Y85M+K90I |
| F88A+L59A+Y89D+N86H+F84C | F88A+L59A+Y89D+N86H+Y85M+K90R |
| F88A+L59A+Y89D+N86H+F84G | F88A+L59A+Y89D+N86H+Y85M+K90T |
| F88A+L59A+Y89D+N86H+F84L | F88A+L59A+Y89D+N86H+Y85M+K90Y |
| F88A+L59A+Y89D+N86H+F84R | F88A+L59A+Y89D+N86H+Y85M+K90M |
| F88A+L59A+Y89D+N86H+F84V | F88A+L59A+Y89D+N86H+Y85M+K90H |
| F88A+L59A+Y89D+N86H+F84P | F88A+L59A+Y89D+N86H+Y85M+K90N |
| F88A+L59A+Y89D+N86H+F84A | F88A+L59A+Y89D+N86H+Y85M+K90P |
| F88A+L59A+Y89D+N86H+F84M | F88A+L59A+Y89D+N86H+Y85M+K90Q |
| F88A+L59A+Y89D+N86H+F84E | F88A+L59A+Y89D+N86H+Y85M+K90W |
| F88A+L59A+Y89D+N86H+F84K | F88A+L59A+Y89D+N86H+Y85M+T91W |
| F88A+L59A+Y89D+N86H+F84W | F88A+L59A+Y89D+N86H+Y85M+T91V |
| F88A+L59A+Y89D+N86H+F84Q | F88A+L59A+Y89D+N86H+Y85M+T91G |
| F88A+L59A+Y89D+N86H+F84T | F88A+L59A+Y89D+N86H+Y85M+T91C |
| F88A+L59A+Y89D+N86H+F84H | F88A+L59A+Y89D+N86H+Y85M+T91R |
| F88A+L59A+Y89D+N86H+F84D | F88A+L59A+Y89D+N86H+Y85M+T91A |
| F88A+L59A+Y89D+N86H+F84Y | F88A+L59A+Y89D+N86H+Y85M+T91F |
| F88A+L59A+Y89D+N86H+F84N | F88A+L59A+Y89D+N86H+Y85M+T91I |
| F88A+L59A+Y89D+N86H+F84I | F88A+L59A+Y89D+N86H+Y85M+T91M |
| F88A+L59A+Y89D+N86H+Y85M | F88A+L59A+Y89D+N86H+Y85M+T91E |
| F88A+L59A+Y89D+N86H+Y85M+K90F | F88A+L59A+Y89D+N86H+Y85M+T91L |
| F88A+L59A+Y89D+N86H+Y85M+K90G | F88A+L59A+Y89D+N86H+Y85M+T91S |
| F88A+L59A+Y89D+N86H+Y85M+K90D | F88A+L59A+Y89D+N86H+Y85M+T91K |
| F88A+L59A+Y89D+N86H+Y85M+K90C | F88A+L59A+Y89D+N86H+Y85M+T91H |
| F88A+L59A+Y89D+N86H+Y85M+K90S | F88A+L59A+Y89D+N86H+Y85M+T91Q |
| F88A+L59A+Y89D+N86H+Y85M+K90A | F88A+L59A+Y89D+N86H+Y85M+T91D |
| F88A+L59A+Y89D+N86H+Y85M+K90E | F88A+L59A+Y89D+N86H+Y85M+T91N |
| F88A+L59A+Y89D+N86H+Y85M+K90L | F88A+L59A+Y89D+N86H+Y85M+T91P |
| F88A+L59A+Y89D+N86H+Y85M+K90V | F88A+L59A+Y89D+N86H+Y85M+T91Y |
| | |
|---|---|
| F88A+L59A+Y89D+N86H+Y85M+K90G+T91M | F88A+L59A+Y89D+N86H+Y85M+K90G+T91M+F409W |
| F88A+L59A+Y89D+N86H+Y85M+K90G+T91M+P83F | F88A+L59A+Y89D+N86H+Y85M+K90G+T91M+F409N |
| F88A+L59A+Y89D+N86H+Y85M+K90G+T91M+P83G | F88A+L59A+Y89D+N86H+Y85M+K90G+T91M+S417F |
| F88A+L59A+Y89D+N86H+Y85M+K90G+T91M+P83D | F88A+L59A+Y89D+N86H+Y85M+K90G+T91M+S417E |
| F88A+L59A+Y89D+N86H+Y85M+K90G+T91M+P83C | F88A+L59A+Y89D+N86H+Y85M+K90G+T91M+S417Y |
| F88A+L59A+Y89D+N86H+Y85M+K90G+T91M+P83S | F88A+L59A+Y89D+N86H+Y85M+K90G+T91M+S417M |
| F88A+L59A+Y89D+N86H+Y85M+K90G+T91M+P83A | F88A+L59A+Y89D+N86H+Y85M+K90G+T91M+S417A |
| F88A+L59A+Y89D+N86H+Y85M+K90G+T91M+P83E | F88A+L59A+Y89D+N86H+Y85M+K90G+T91M+S417Q |
| F88A+L59A+Y89D+N86H+Y85M+K90G+T91M+P83L | F88A+L59A+Y89D+N86H+Y85M+K90G+T91M+S417C |
| F88A+L59A+Y89D+N86H+Y85M+K90G+T91M+P83V | F88A+L59A+Y89D+N86H+Y85M+K90G+T91M+S417L |
| F88A+L59A+Y89D+N86H+Y85M+K90G+T91M+P83I | F88A+L59A+Y89D+N86H+Y85M+K90G+T91M+S417I |
| F88A+L59A+Y89D+N86H+Y85M+K90G+T91M+P83R | F88A+L59A+Y89D+N86H+Y85M+K90G+T91M+S417T |
| F88A+L59A+Y89D+N86H+Y85M+K90G+T91M+P83T | F88A+L59A+Y89D+N86H+Y85M+K90G+T91M+S417V |
| F88A+L59A+Y89D+N86H+Y85M+K90G+T91M+P83Y | F88A+L59A+Y89D+N86H+Y85M+K90G+T91M+S417W |
| F88A+L59A+Y89D+N86H+Y85M+K90G+T91M+P83M | F88A+L59A+Y89D+N86H+Y85M+K90G+T91M+S424N |
| F88A+L59A+Y89D+N86H+Y85M+K90G+T91M+P83H | F88A+L59A+Y89D+N86H+Y85M+K90G+T91M+S424F |
| F88A+L59A+Y89D+N86H+Y85M+K90G+T91M+P83N | F88A+L59A+Y89D+N86H+Y85M+K90G+T91M+S424T |
| F88A+L59A+Y89D+N86H+Y85M+K90G+T91M+P83K | F88A+L59A+Y89D+N86H+Y85M+K90G+T91M+S424G |
| F88A+L59A+Y89D+N86H+Y85M+K90G+T91M+P83Q | F88A+L59A+Y89D+N86H+Y85M+K90G+T91M+S424I |
| F88A+L59A+Y89D+N86H+Y85M+K90G+T91M+P83W | F88A+L59A+Y89D+N86H+Y85M+K90G+T91M+S424V |
| F88A+L59A+Y89D+N86H+Y85M+K90G+T91M+F409Y | F88A+L59A+Y89D+N86H+Y85M+K90G+T91M+S424A |
| F88A+L59A+Y89D+N86H+Y85M+K90G+T91M+F409I | F88A+L59A+Y89D+N86H+Y85M+K90G+T91M+S424L |
| F88A+L59A+Y89D+N86H+Y85M+K90G+T91M+F409G | F88A+L59A+Y89D+N86H+Y85M+K90G+T91M+V234A |
| F88A+L59A+Y89D+N86H+Y85M+K90G+T91M+F409F | F88A+L59A+Y89D+N86H+Y85M+K90G+T91M+L380A |
| F88A+L59A+Y89D+N86H+Y85M+K90G+T91M+F409A | F88A+L59A+Y89D+N86H+Y85M+K90G+T91M+C61A |
| F88A+L59A+Y89D+N86H+Y85M+K90G+T91M+F409L | F88A+L59A+Y89D+N86H+Y85M+K90G+T91M+P83S |
| |
|---|
| F88A+L59A+Y89D+N86H+Y85M+K90G+T91M+P83S+V234A |
| F88A+L59A+Y89D+N86H+Y85M+K90G+T91M+P83S+V234A+L380A |
| F88A+L59A+Y89D+N86H+Y85M+K90G+T91M+P83S+V234A+L380A+S417A |
| F88A+L59A+Y89D+N86H+Y85M+K90G+T91M+P83S+V234A+L380A+S417C |
| F88A+L59A+Y89D+N86H+Y85M+K90G+T91M+P83S+V234A+L380A+S417D |
| F88A+L59A+Y89D+N86H+Y85M+K90G+T91M+P83S+V234A+L380A+S417E |
| F88A+L59A+Y89D+N86H+Y85M+K90G+T91M+P83S+V234A+L380A+S417F |
| F88A+L59A+Y89D+N86H+Y85M+K90G+T91M+P83S+V234A+L380A+S417G |
| F88A+L59A+Y89D+N86H+Y85M+K90G+T91M+P83S+V234A+L380A+S417H |
| F88A+L59A+Y89D+N86H+Y85M+K90G+T91M+P83S+V234A+L380A+S417I |
| F88A+L59A+Y89D+N86H+Y85M+K90G+T91M+P83S+V234A+L380A+S417K |
| F88A+L59A+Y89D+N86H+Y85M+K90G+T91M+P83S+V234A+L380A+S417L |
| F88A+L59A+Y89D+N86H+Y85M+K90G+T91M+P83S+V234A+L380A+S417M |
| F88A+L59A+Y89D+N86H+Y85M+K90G+T91M+P83S+V234A+L380A+S417N |
| F88A+L59A+Y89D+N86H+Y85M+K90G+T91M+P83S+V234A+L380A+S417P |
| F88A+L59A+Y89D+N86H+Y85M+K90G+T91M+P83S+V234A+L380A+S417Q |
| F88A+L59A+Y89D+N86H+Y85M+K90G+T91M+P83S+V234A+L380A+S417R |
| F88A+L59A+Y89D+N86H+Y85M+K90G+T91M+P83S+V234A+L380A+S417T |
| F88A+L59A+Y89D+N86H+Y85M+K90G+T91M+P83S+V234A+L380A+S417V |
| F88A+L59A+Y89D+N86H+Y85M+K90G+T91M+P83S+V234A+L380A+S417W |
| F88A+L59A+Y89D+N86H+Y85M+K90G+T91M+P83S+V234A+L380A+S417Y |
| F88A+L59A+Y89D+N86H+Y85M+K90G+T91M+P83S+V234A+L380A+S417A+F409L |
| F88A+L59A+Y89D+N86H+Y85M+K90G+T91M+P83S+V234A+L380A+S417A+S424A |
| F88A+L59A+Y89D+N86H+Y85M+K90G+T91M+P83S+V234A+L380A+S417A+S424G |
| F88A+L59A+Y89D+N86H+Y85M+K90G+T91M+P83S+V234A+L380A+S417A+K167I |
| F88A+L59A+Y89D+N86H+Y85M+K90G+T91M+P83S+V234C+L380A+S417A |
| F88A+L59A+Y89D+N86H+Y8SM+K90G+T91M+P83S+V234A+L380A+S417I+A168E |
| F88A+L59A+Y89D+N86H+Y85M+K90G+T91M+P3S+V234A+L380A+S417I+A168E+A379L+S424A |
| F88A+L59A+Y89D+N86H+Y85M+K90G+T91M+P3S+V234A+L380A+S417I+A168E+A379L+S424G |
| F88A+L59A+Y89D+N86H+Y85M+K90G+T91M+P3S+V234C+L380A+S417I+A168E+A379L+S424A |
| F88A+L59A+Y89D+N86H+Y85M+K90G+T91M+P3S+V234A+L380A+S417I+A168E+A379L+S424A+F301S |
| F88A+L59A+Y89D+N86H+Y85M+K90G+T91M+P3S+V234A+L380A+S417I+A168E+A379L+S424A+F364C |
| F88A+L59A+Y89D+N86H+Y85M+K90G+T91M+P3S+V234A+L380A+S417I+A168E+A379L+S424A+T452A |
| F88A+L59A+Y89D+N86H+Y85M+K90G+T91M+P3S+V234A+L380A+S417I+A168E+A379L+S424A+K358R+F449L |
| F88A+L59A+Y89D+N86H+Y85M+K90G+T91M+P3S+V234A+L380A+S417I+A168E+A379L+S424A+M180V |
| F88A+L59A+Y89D+N86H+Y85M+K90G+T91M+P3S+V234A+L380A+S417I+A168E+A379L+S424A+G164S |
| F88A+L59A+Y89D+N86H+Y85M+K90G+T91M+P3S+V234A+L380A+S417I+A168E+A379L+S424A+F301S+G164S |
| F88A+L59A+Y89D+N86H+Y85M+K90G+T91M+P3S+V234A+L380A+S417I+A168E+A379L+S424A+F301S+M180V |
| F88A+L59A+Y89D+N86H+Y85M+K90G+T91M+P3S+V234A+L380A+S417I+A168E+A379L+S424A+F301S+F449L |
| F88A+L59A+Y89D+N86H+Y85M+K90G+T91M+P3S+V234A+L380A+S417I+A168E+A379L+S424A+F301S+F409L |
| F88A+L59A+Y89D+N86H+Y85M+K90G+T91M+P3S+V234A+L380A+S417I+A168E+A379L+S424A+F301S+G164S+T4 52S |
| F88A+L59A+Y89D+N86H+Y85M+K90G+T91M+P3S+V234A+L380A+S417I+A168E+A379L+S424A+F301S+G164S+F4 49L |
| F88A+L59A+Y89D+N86H+Y85M+K90G+T91M+P3S+V234A+L380A+S417I+A168E+A379L+S424A+F301S+G164S+M 180V |
| F88A+L59A+Y89D+N86H+Y85M+K90G+T91M+P3S+V234A+L380A+S417I+A168E+A379L+S424A+F301S+G164S+M 180V+E171V |
| F88A+L59A+Y89D+N86H+Y85M+K90G+T91M+P3S+V234A+L380A+S417I+A168E+A379L+S424A+F301S+G164S+F4 49L+L453V |
| F88A+L59A+Y89D+N86H+Y85M+K90G+T91M+P3S+V234A+L380A+S417I+A168E+A379L+S424A+F301S+G164S+K1 67I |
| F88A+L59A+Y89D+N86H+Y85M+K90G+T91M+P3S+V234A+L380A+S417I+A168E+A379L+S424A+F301S+G164S+T4 52S+M180V |
| F88A+L59A+Y89D+N86H+Y85M+K90G+T91M+P3S+V234A+L380A+S417I+A168E+A379L+S424A+F301S+G164S+T4 52S+F449L |
| F88A+L59A+Y89D+N86H+Y85M+K90G+T91M+P3S+V234A+L380A+S417I+A168E+A379L+S424A+F301S+G164S+T4 52S+M180V+E171V |
| F88A+L59A+Y89D+N86H+Y85M+K90G+T91M+P3S+V234A+L380A+S417I+A168E+A379L+S424A+F301S+G164S+T4 52S+M180V+F449L |
| F88A+L59A+Y89D+N86H+Y85M+K90G+T91M+P3S+V234A+L380A+S417I+A168E+A379L+S424A+F301S+G164S+T4 52S+M180V+F320M |
| F88A+L59A+Y89D+N86H+Y85M+K90G+T91M+P3S+V234A+L380A+S417I+A168E+A379L+S424A+F301S+G164S+T4 52S+M180V+F320H |
| F88A+L59A+Y89D+N86H+Y85M+K90G+T91M+P3S+V234A+L380A+S417I+A168E+A379L+S424A+F301S+G164S+T4 52S+M180V+Y322N |
| F88A+L59A+Y89D+N86H+Y85M+K90G+T91M+P3S+V234A+L380A+S417I+A168E+A379L+S424A+F301S+G164S+T4 52S+M180V+Y322T |
| F88A+L59A+Y89D+N86H+Y85M+K90G+T91M+P3S+V234A+L380A+S417I+A168E+A379L+S424A+F301S+G164S+T4 52S+M180V+F449L+F320H+Y322C |
| F88A+L59A+Y89D+N86H+Y85M+K90G+T91M+P3S+V234A+L380A+S417I+A168E+A379L+S424A+F301S+G164S+T4 52S+M180V+F449L+F320H+Y322T |
| F88A+L59A+Y89D+N86H+Y85M+K90G+T91M+P3S+V234A+L380A+S417I+A168E+A379L+S424A+F301S+G164S+T4 52S+M180V+F449L+F320H+Y322S |
| F88A+L59A+Y89D+N86H+Y85M+K90G+T91M+P3S+V234A+L380A+S417I+A168E+A379L+S424A+F301S+G164S+T4 52S+M180V+F449L+Y322S |
| F88A+L59A+Y89D+N86H+Y85M+K90G+T91M+P3S+V234A+L380A+S417I+A168E+A379L+S424A+F301S+G164S+T4 52S+M180V+F449L+Y322T |
| F88A+L59A+Y89D+N86H+Y85M+K90G+T91M+P3S+V234A+L380A+S417I+A168E+A379L+S424A+F301S+G164S+T4 52S+M180V+F449L+F320H +Y322T+M91I |
| F88A+L59A+Y89D+N86H+Y85M+K90G+T91M+P3S+V234A+L380A+S417I+A168E+A379L+S424A+F301S+G164S+T4 52S+M180V+F449L+F320H +Y322T+D315V |
or
(2) a mutant obtained from a mutation of conserved amino acids of a wild-type transaminase, with the SEQ ID NO: 1 of the wild-type CvTA transaminase as a reference, the conserved amino acids have at least any one of the following combination mutations: (a)L59A+F88A; (b)L59A+F88A+F89D; (c)L59A+F88A+F89D+N86H; (d)L59A+F88A+F89D+N86H +Y85M;
wherein the wild-type transaminase is selected from any one of the following numbered from 1 to 54:

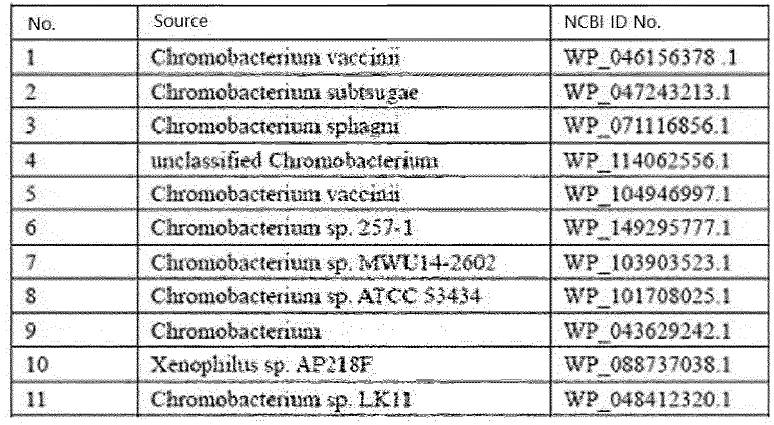

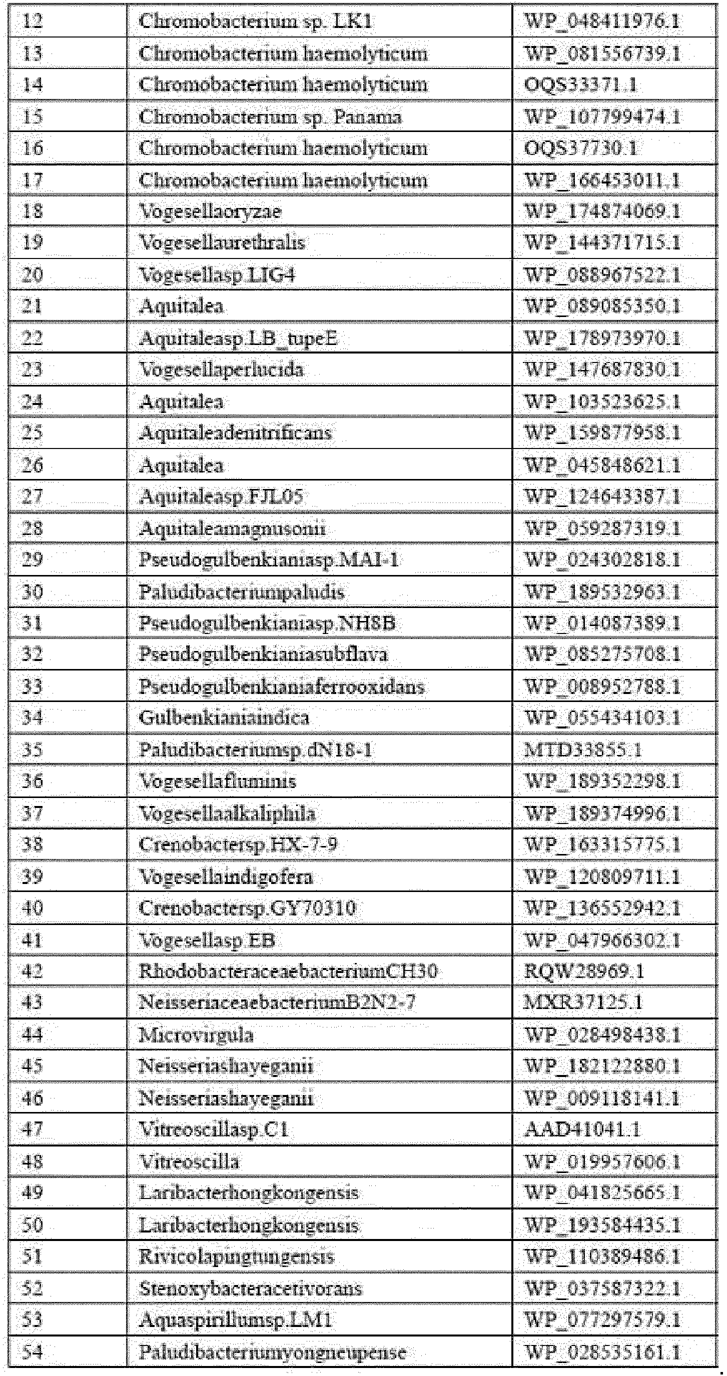

2. A DNA molecule encoding the transaminase mutant according to claim 1.

3. A recombinant plasmid, wherein the recombinant plasmid is ligated with the DNA molecule according to claim 2.

4. A method for synthesizing a chiral amine compound, comprising:
performing a transamination reaction with the transaminase mutant according to claim 1 on a ketone substrate as shown in formula I under the action of an amino donor; and obtaining the chiral amine compound; 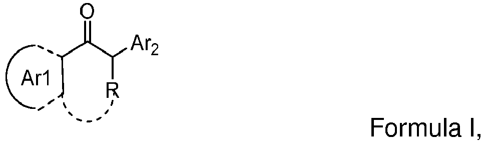
wherein Ar1 represents a substituted or unsubstituted aryl, a substituted or unsubstituted arylene, or a substituted or unsubstituted heteroarylene;
Ar2 represents a substituted or unsubstituted aryl, or a substituted or unsubstituted cycloalkyl; and
optionally R represents a substituted or unsubstituted C1 -C5 alkylene, and the R is connected to Ar1 or Ar2 to form a ring;
wherein a substituent in the substituted aryl, the substituted aryl arylene, the substituted heteroarylene or the substituted alkylene is independently selected from halogen, hydroxy, or amino, and a heteroatom in the substituted heteroarylene is selected from N, O or S.

5. The synthesis method according to claim 4, wherein the substituent in the substituted aryl or the substituted arylene is a halogen or -S-CH₃, and the halogen or -S-CH₃ is located in any one or more positions of ortho, meta or para position of the aryl or arylene.

6. The synthesis method according to claim 5, wherein the halogen is F, Cl or Br.

7. The synthesis method according to claim 4, wherein the ketone substrate as shown in formula I is selected from any one of the following:
i) the Ar1 represents a substituted or unsubstituted aryl, or a substituted or unsubstituted arylene, the Ar2 represents an unsubstituted aryl, and the R represents an unsubstituted C1~C5 alkylene, and the R is connected to Ar1 to form a ring; or
ii) the Ar1 represents a unsubstituted heteroarylene, the Ar2 represents a halogen-substituted aryl, and the R represents a hydroxyl-substituted C1~C5 alkylene, and the R is connected to Ar1 to form a ring; or
iii) the Ar1 represents a halogen- and amino-substituted arylene, and the Ar2 represents a C3-C8 cycloalkyl; or
iv) the Ar1 represents an unsubstituted cycloalkyl or an unsubstituted aryl, and the Ar2 represents a substituted or unsubstituted cycloalkyl, or a substituted or unsubstituted aryl;
v) the Ar1 represents a hydroxyl-, a methyl-, an ethyl-, or a -S-CH₃-substituted cycloalkyl, or a hydroxyl-, a methyl-, an ethyl-, or a -S-CH₃-substituted aryl, and the Ar2 represents an unsubstituted aryl or an unsubstituted alkyl.

8. The synthesis method according to claim 7, wherein the ketone substrate is selected from the group consisting of:

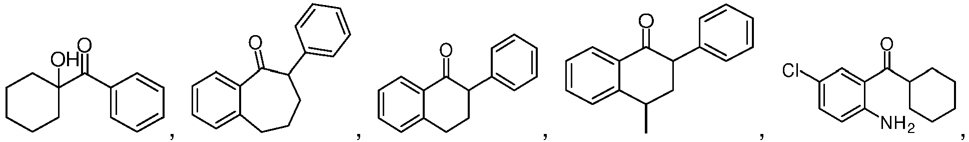

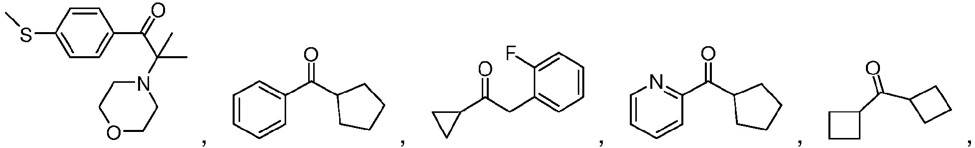

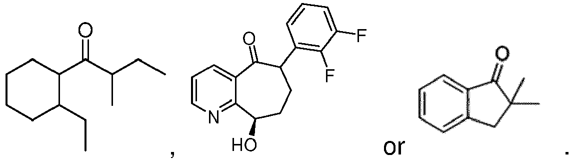

9. The synthesis method according to claim 4, wherein the amino donor is selected from the group consisting of isopropylamine, isopropylamine hydrochloride, alanine, n-butylamine or aniline.
